# EUROPEAN PATENT SPECIFICATION

(11) **EP 3 124 600 B1**
(45) Date of publication and mention of the grant of the patent: **29.12.2021**
(21) Application number: 15754683.9
(22) Date of filing: 26.02.2015
(51) Int. Cl.: C12N 5/071, A61L 27/00, C12M 3/00, C12N 5/0735, C12N 5/077, C12N 5/0775, C12N 5/10

(54) **METHOD FOR GENERATING A CELL CONDENSATE FOR SELF-ORGANISATION**
VERFAHREN ZUR HERSTELLUNG EINES ZELLKONDENSATS ZUR SELBSTORGANISATION
ROCÉDÉ DE FABRICATION D'UN CONDENSÉ CELLULAIRE DANS L'OBJECTIF DE SON ORGANISATION AUTOMATIQUE

(30) Priority: 27.02.2014 JP 2014037341
(43) Date of publication of application: 01.02.2017
(73) Proprietor: Public University Corporation Yokohama City University, Yokohama-shi, Kanagawa 236-0027 (JP)
(72) Inventor: TAKEBE, Takanori, Yokohama-shi Kanagawa 236-0004 (JP); TANIGUCHI, Hideki, Yokohama-shi Kanagawa 236-0004 (JP); YOSHIKAWA, Hiroshi, Osaka 565-0871 (JP)
(74) Representative: J A Kemp LLP
(86) International application number: PCT/JP2015/055695
(87) International publication number: WO 2015/129822

(56) References cited:
- EP-A1- 2 762 558
- EP-A1- 2 774 983
- WO-A1-2012/119074
- WO-A1-2013/047639
- US-A1- 2010 166 713
- US-A1- 2013 252 337
- S YOSHIDA ET AL.: "Generation of stratified squamous epithelial progenitor cells from mouse induced pluripotent stem cells", PLOS ONE, vol. 6, no. 12, December 2011 (2011-12), pages 1-10, XP55326521, Public Library of Science ISSN: 1932-6203
- TAKEBE T. ET AL.: 'Generation of a vascularized and functional human liver from an iPSC-derived organ bud transplant' NAT.PROTOC. vol. 9, no. 2, 2014, pages 396 - 409, XP055166485
- ZHANG M. ET AL.: 'Rapid and efficient generation of neurons from human pluripotent stem cells in a multititre plate format' J.VIS.EXP. vol. 5, no. 73, 2013, page E4335, XP055221183
- ISENBERG B.C. ET AL.: 'Vascular smooth muscle cell durotaxis depends on substrate stiffness gradient strength' BIOPHYS.J. vol. 9 7, no. 5, 2009, pages 1313 - 22, XP055221184
- CHAWLA K. ET AL.: 'Biodegradable and biocompatible synthetic saccharide-Peptide hydrogels for three-dimensional stem cell culture' BIOMACROMOLECULES vol. 12, no. 3, 2011, pages 560 - 567, XP055221190
- FISCHER R.S. ET AL.: 'Stiffness-controlled three-dimensional extracellular matrices for high-resolution imaging of cell behavior' NAT.PROTOC. vol. 7, no. 11, 2012, pages 2056 - 66, XP055221192
- OWEN S.C. ET AL.: 'Design of three-dimensional biomimetic scaffolds' J.BIOMED.MATER.RES.A vol. 94, no. 4, 2010, pages 1321 - 31, XP055221201
- WANG L.S. ET AL.: 'Enzymatically cross-linked gelatin-phenol hydrogels with a broader stiffness range for osteogenic differentiation of human mesenchymal stem cells' ACTA BIOMATER. vol. 8, no. 5, 2012, pages 1826 - 37, XP028476153
- KUBOKI T. ET AL.: '2D-DIGE proteomic analysis of mesenchymal stem cell cultured on the elasticity-tunable hydrogels' CELL STRUCT. FUNCT. vol. 37, no. 2, 2012, pages 127 - 139, XP055357951
- HIDEKI TANIGUCHI ET AL.: 'Kansaibo to Bisho Kankyo no Sogo Sayo ni Motozuku Hito Sanjigen Soshiki no Jin'iteki Saikosei' MOLECULAR TARGETED THERAPY FOR CANCER vol. 12, no. 3, 2014, pages 66 - 71, XP008184611
- TAKEBE T. ET AL.: 'Vascularized and Complex Organ Buds from Diverse Tissues via Mesenchymal Cell -Driven Condensation' CELL STEM CELL vol. 16, no. 5, 2015, pages 556 - 565, XP055329771
- MACQUEEN L., SUN Y. & SIMMONS C.A.: "Mesenchymal stem cell mechanobiology and emerging experimental platforms", J. ROYAL SOC. INTERFACE, vol. 10, 20130179, 2013,
- TAKANORI TAKEBE ET AL: "Vascularized and Complex Organ Buds from Diverse Tissues via Mesenchymal Cell-Driven Condensation", CELL STEM CELL, vol. 16, no. 5, 7 May 2015 (2015-05-07), pages 556-565, XP055329771, AMSTERDAM, NL ISSN: 1934-5909, DOI: 10.1016/j.stem.2015.03.004
- ZAARI N. ET AL.: "Photopolymerization in MicrofluidicGradient Generators: MicroscaleControl of Substrate Compliance toManipulate Cell Response", ADV. MATER., vol. 16, no. 23-24, 17 December 2004 (2004-12-17), pages 2133-2137,
- IVASCU A. & KUBBIES M.: "Rapid Generation of Single-Tumor Spheroids forHigh-Throughput Cell Function and Toxicity Analysis", J. BIOL. SCREENING, vol. 11, no. 8, 2006, pages 922-932,
- ENGLER A.J. ET AL.: "Matrix Elasticity Directs StemCell Lineage Specification", CELL, vol. 126, 25 August 2006 (2006-08-25), pages 677-689,
- SLATER K.: "Tuning the Elastic Moduli of Corning® Matrigel® and Collagen I 3D Matrices by Varying the Protein Concentration", 2017, CORNING LIFE SCIENCES, Tewksbury MA
- TAKEBE T. ET AL.: "Massive and Reproducible Production of LiverBuds Entirely from Human Pluripotent Stem Cells", CELL REPORTS, vol. 21, 5 December 2017 (2017-12-05), pages 2661-2670,
- SAMUEL R. ET AL.: "Generation of functionally competent and durableengineered blood vessels from human inducedpluripotent stem cells", PNAS, vol. 110, no. 31, 30 July 2013 (2013-07-30), pages 12744-12779,
- AU P. ET AL.: "Bone marrow-derived mesenchymal stem cells facilitate engineering of long-lasting functional vasculature", BLOOD, vol. 111, no. 9, 1 May 2008 (2008-05-01), pages 4551-4558,
- KOBAYASHI S. ET AL.: "Reconstruction of human elastic cartilage by a CD44+CD90+ stem cell in the ear perichondrium", PNAS, vol. 108, no. 35, 30 August 2011 (2011-08-30), pages 14479-14484,
- SHEA C.M. ET AL.: "BMP Treatment of C3H10T1/2 Mesenchymal Stem CellsInduces Both Chondrogenesis and Osteogenesis", J. CELL. BIOCHEM., vol. 90, 2003, pages 1112-1117,

## Description

### TECHNICAL FIELD

The present invention relates to a method of preparing a cell condensate for self-organization. More specifically, the present invention relates to a method of preparing a cell condensate that is necessary for directing self-organization into a tissue or an organ of interest.

### BACKGROUND ART

Recently, methods using the self-organization capacity of cells which they inherently possess have been attracting attention as methods of forming tissues/organs with complex structures (Non-Patent Documents Nos. 1 and 2). Self-organization is a process in which one or a few elements construct complex higher structures by exerting intrinsic properties of their own without receiving specific "instructions" (information) from the outside. For example, natural phenomena in which spontaneous order arises from patternless aggregates to form patterns, as in crystallization of snow, are observed. Self-organization is also used in the field of engineering, e.g. in nanotechnology or in preparing optical crystals.

For inducing self-organization, it is necessary to form an aggregate consisting of homogeneous cells in a high-density environment. Studies have been reported in which aggregates were prepared from cultured ES/iPS cells to generate brain, optic cup, pituitary gland, teeth, etc. (Non-Patent Documents Nos. 3 to 7.).

As a technique for preparing such aggregates, a method is mainly used in which tissues of several hundred µm level are formed from cell aggregates of a small number of cells (about several thousand) by using a substrate such as a 96-well plate with U- or V-shaped bottoms that permits cells to gather in the bottom. However, it has been difficult to achieve formation of larger size (200 µm or more) cell condensates from a large number of cells (several ten thousand to several million cells). Therefore, it has been difficult to apply conventional methods to preparation of cell aggregates consisting of diverse cells.

Under these circumstances, it has been desired to develop a self-organization based technique for preparing cell condensates for generating large and complex tissues/organs (as from humans) compared to tissues/organs of small animals like mouse.

### PRIOR ART LITERATURE

### Non-Patent Documents

Non-Patent Document No. 1: Camazine, S., Deneubourg, J. -L., Franks, N. R., Sneyd, J., Theraulaz, G. & Bonabeau, E. Self-Organization in Biological Systems (Princeton Univ. Press, 2001).
Non-Patent Document No. 2: Takeichi, M. Self-organization of animal tissues: cadherin-mediated processes. Dev. Cell 21, 24-26 (2011).
Non-Patent Document No. 3: Eiraku, E. et al. Self-organizing optic-cup morphogenesis in three-dimensional culture. Nature 472, 51-56 (2011).
Non-Patent Document No. 4: Eiraku, M. et al. Self-organized formation of polarized cortical tissues from ESCs and its active manipulation by extrinsic signals. Cell Stem Cell 3, 519-532 (2008).
Non-Patent Document No. 5: Suga, H. et al. Self-formation of functional adenohypophysis in three-dimensional culture. Nature 480, 57-62 (2011).
Non-Patent Document No. 6: Sato, T. et al. Single Lgr5 stem cells build crypt-villus structures in vitro without a mesenchymal niche. Nature 459, 262-265 (2009).
Non-Patent Document No 7: Takebe T. et al. Generation of a vascularized and functional human liver from an iPSC-derived organ bud transplant. Nature Protocols 9, no 2, 396-409 (2014).

### DISCLOSURE

### Problem to be solved.

There is a need for a platform technology which recapitulates interactions among organ cells, vascular cells and mesenchymal cells that are essential for early processes of organogenesis, to thereby induce 3D organ primordia (starting material for organs) and enable generation of vascularized functional organs (Nature, 499 (7459), 481-484; PCT/JP2012/074840 Method for Preparing Tissue and Organ). Significantly, for the development of drugs or realization of regenerative medicine for diseases in kidney, liver, lung, etc., it is essential to recapitulate three-dimensional complex structures (integrating not only a vasculature but also higher structures such as ureteral structure, biliary structure, tracheal structure, etc.) and cell polarity. Moreover, induction of an organ of interest is achieved through interactions with other organs.

Therefore, in order to maximize the function of tissues induced from pluripotent stem cells or tissues isolated from individuals, three-dimensional tissue constructs should be formed which enable reconstitution of continuity with diverse higher structures and other organs. According to conventionally devised methods, however, only tissue constructs having a vascular structure alone have been prepared from the three types of cells or tissues. No technique has been invented for preparing more complex, higher structures (such as ureteral structure, biliary structure and tracheal structure).

It is an object of the present invention to find out the requirements necessary for preparing a cell condensate *in vitro* from a large number of cells (several ten thousand to several million cells). It is another object of the present invention to provide a method of forming a cell condensate for self-organization which is capable of realizing complex higher structures (such as liver and kidney) and interactions with other organs.

### Means to solve the problem

The present inventors have established a three-dimensional culture technique using spatiotemporal interactions of three different cell lineages; this technique has realised "directed differentiation of organ cells based on reconstitution of organs". With this technique, they have succeeded in preparing three-dimensional tissues/organs having complex higher structures from isolated, multiple types of cells or tissues by the operations 1 to 4 described below.

### 1. Preparation of Necessary Cells/Tissues

A) Cells/tissues of a desired type or types that are necessary for self-organization into tissues with complex structures are prepared. The types or numbers to be combined do not matter.
B) A mixture in solution that consists of a desired type or types of cells/tissues in a total number of approximately 2 million is mixed with approximately 100,000 to 400,000 isolated mesenchymal cells.

### 2. Preparation of Support

A) A support with an appropriate stiffness is formed and solidified on a cell culture dish. Preferable materials for the support include, but are not limited to, hydrogels (such as polyacrylamide gel).
B) Chemical/physical modifications are provided on the prepared support. Giving such modifications, however, is not an essential requirement. Preferable chemical factors include, but are not limited to, Matrigel and laminin.
C) The stiffness of the support need not be uniform and may vary depending on the shape, size and quantity of a condensate of interest. The stiffness of the support may be provided with aspatial/temporal gradient or patterned, for use in subsequent experiments.

### 3. Preparation and Culture of Cell Condensates

A) The cell/tissue mixture in solution as prepared in 1 above is plated on the support prepared in 2 above to form condensates. The thus formed condensates may be cultured for an elongated period so that it can be used for self-organization into organs of interest *in vitro.*

By combining mesenchymal cells with a culture substrate that permits cells to gather in the bottom, condensates can also be prepared from the cells if they are small in number.

### 4. Transplantation of Cell Condensates

By subjecting the condensates prepared in 3 above to long-term culture or transplanting them into living bodies to induce blood perfusion and allow self-organization into higher tissues with a complex structure, tissues/organs can be prepared that have a highly ordered tissue structure comparable to that of adult tissues.

The above-described technique which prepares a complex cell condensate consisting of cells of a desired type or types by combining mesenchymal cells with physicochemical properties of a support has not existed to date.

### Summary of the invention.

As recited in the appended claims, the invention concerns:
(1) A method of preparing a cell condensate *in vitro,* comprising culturing a mixture of cells and/or tissues of a desired type and undifferentiated mesenchymal cells to form a cell condensate wherein the mixture of cells and/or tissues of a desired type and undifferentiated mesenchymal cells is cultured on a gel-like support on which the undifferentiated mesenchymal cells are capable of contraction, wherein the gel-like support has a stiffness of a Young's modulus of 1-200 kPa, and wherein the cell condensate is formed autonomously.
(2) The method of (1) above, wherein the culture is two-dimensional culture.
(3) The method of (1) or (2) above, wherein the gel-like support is planar or the side of the gel-like support on which culture is performed has a U- or V-shaped cross-section.
(4) The method of any one of (1) to (3) above, wherein the gel-like support is planar and the stiffness of the central part of the gel-like support is greater than the stiffness of the peripheral part thereof.
(5) The method of any one of (1) to (3) above, wherein the gel-like support is planar and the stiffness of the peripheral part of the gel-like support is greater than the stiffness of the central part thereof.
(6) The method of any one of (1) to (3) above, wherein the gel-like support is planar and patterned and has one or more patterns in which the stiffness of the central part is greater than the stiffness of the peripheral part.
(7) The method of any one of (1) to (3) above, wherein the gel-like support is planar and patterned and has one or more patterns in which the stiffness of the peripheral part is greater than the stiffness of the central part.
(8) The method of any one of (1) to (7) above, wherein the size of the cell condensate is 1 mm or more.
(9) The method of any one of (1) to (8) above, wherein the mixture of cells and/or tissues of a desired type and undifferentiated mesenchymal cells is cultured without using scaffold materials.
(10) The method of any one of (1) to (9) above, wherein the cells and/or tissues mixed with the undifferentiated mesenchymal cells are derived from liver, pancreas, intestine, lung, kidney, heart, brain or cancer.
(11) The method of any one of (1) to (9) above, wherein the cells mixed with the undifferentiated mesenchymal cells are pluripotent cells.
(12) The method of any one of (1) to (9) above, wherein the tissues mixed with the undifferentiated mesenchymal cells are tissues induced from pluripotent cells.
(13) The method of (11) or (12) above, wherein the pluripotent cell is a pluripotent cell obtained by induction from reprogramming.
(14) The method of any one of (1) to (13) above, wherein the cells and/or tissues of a desired type have a total cell count of 400,000 or more and the undifferentiated mesenchymal cells are 100,000 to 400,000 in number.
(15) The method of any one of (1) to (14) above, wherein the gel-like support has a stiffness of a Young's modulus of 1-100kPa.
(16) The method of (15) above, wherein the gel-like support has a stiffness of a Young's modulus of 1-50kPa.
(17) A method of preparing a three-dimensional tissue structure, said method comprising the method of any one of (1) to (16) above and further comprising allowing self-organization of the cell condensate.

### Applications

As described herein, a cell condensate of theoretically any complex composition can be formed by combining a mesenchymal stem cell and a support or a substrate that will allow cells to gather in the bottom. Accordingly, tissues and organs can be constructed without using scaffolds.

First, the cell condensate is expected to find use as an artificial constitution system for more complex tissues and organs. For example, with the cell condensate it may be possible to prepare three-dimensional complex structures that are provided with not only a vascular network but also higher structures such as ureteral structure, biliary structure, tracheal structure, etc. Further, a great number of organs essentially require that reconstitution associated with other organs be realized in order to exhibit their functions; e.g., in liver, reconstitution of junctions with bile duct and pancreatic duct and connection to duodenum is essential for exhibiting its function. Thus, a cell condensate which recapitulates interactions with other organs is prepared. This cell condensate is expected to find use as a system for inducing self-organization into complex organs existing in the body.

Secondly, since an inexpensive and comparatively easy-to-process support is used, its industrial applicability toward mass production of tissues is high. Mass production of tissues of a desired shape, size and number can be realized at low cost by combining the cell condensate with the multi-patterning of the support or other techniques.

The technique of generating a 3D tissue construct self-organized from a cell condensate prepared from stem cells such as iPS cells is applicable to generation of human functional cells which has been difficult to achieve to date; transplantation of tissues and organs; screening in drug discovery; a novel analysis system for evaluating the relationships between development of drug effects and supporting tissues (blood vessels, nerves, stroma, etc.) and so on.

### BRIEF DESCRIPTION OF THE DRAWINGS

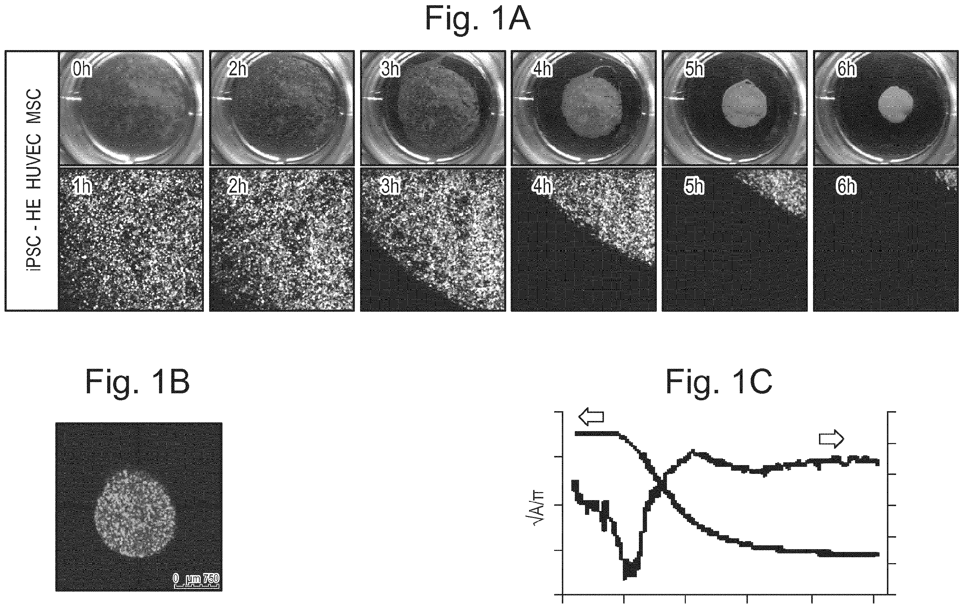

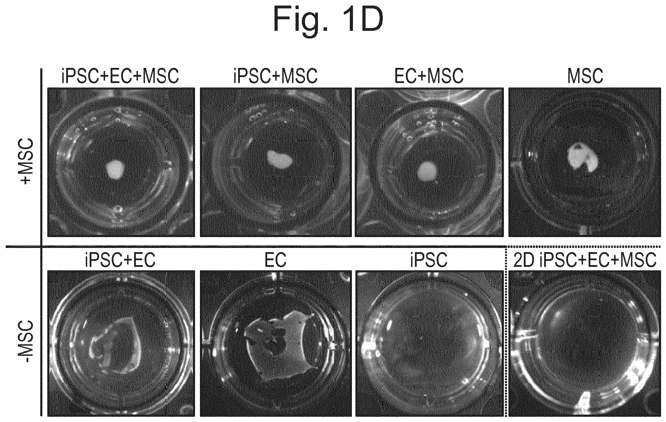

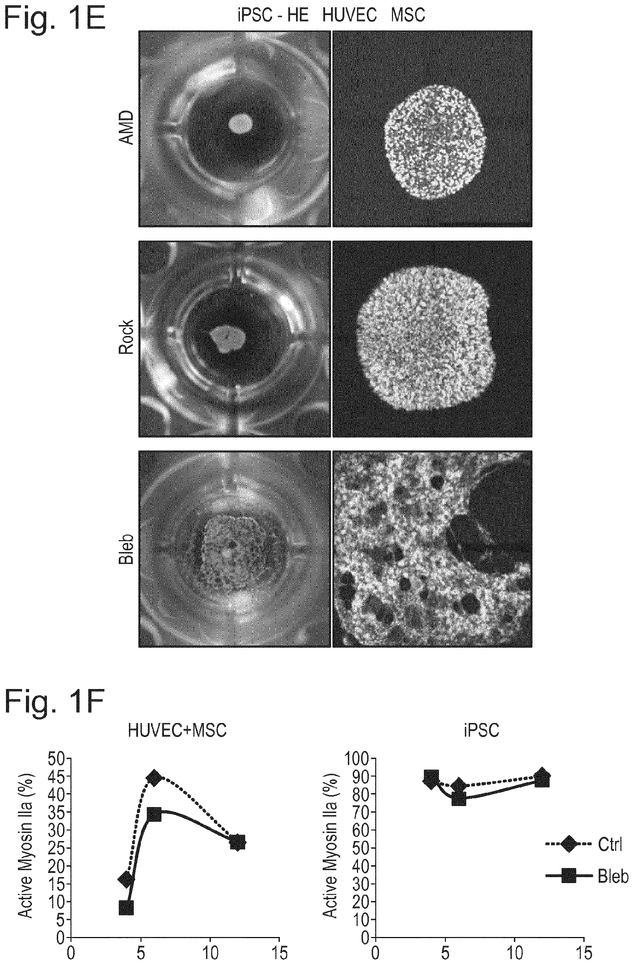

[Fig. 1] Preparation of cell condensates via contraction of mesenchymal cells
   (A) Time-dependent changes in the process of formation of cell condensates. (Green) iPSC-hepatic endoderm cells; (Light red) human vascular endothelial cells; (Colorless) mesenchymal cells.
   (B) Formation of self-organized, iPSC or iPS cell-derived liver buds
   (C) Temporal development of dynamics of cell condensate formation. (Red) square root of the projected area of condensate. This can be used as an indicator showing the location of the edge of condensate. After about 13 hr, an exponential function provides good approximation (black dotted line). ; (Blue) circularity of condensate calculated from the projected area and the contour line length of condensate.
   (D) Necessity of mesenchymal cells in cell condensate formation
   (E) Inhibitory experiment against cell condensate formation process using various chemical substances.
   (F) Time-dependent changes in the content of active form of myosin and the inhibition thereof
[Fig. 2] Optimization of stiffness environment in cell condensate formation (A,B) Cell condensate formation experiments under various stiffness conditions. (A) Macroscopic observation after 48 hr of culture. (B) Time-dependent changes in cell movement under confocal laser microscope. (C-G) Characterization of MSCs in cell condensate. trajectories (C); time dependency of velocity and order parameter (D, E); and dependency on substrate stiffness (F, G).
[Fig. 3] Experiments on the formation of condensates for self-organization using diverse tissue-derived cells
   (A,B) Cell condensate formation using pancreatic β cells (A) and self-organization (B). (C,D) Cell condensate formation experiments using other organ cells/tissues.
[Fig. 4] *In vivo* self-organization of diverse tissue-derived cell condensates and development of their function
   (A) Functional vascularization occurs in 2 to 3 days after transplantation.
   (B) Comparison between the conventional and invention methods of the time required for blood perfusion.
   (C) Direct anastomosis of mouse and human blood vessels.
   (D) Glomeruli and renal tubules formed by cell condensates prepared from embryonic renal cells.
   (E) Islet-like tissues formed by cell condensates prepared from β cells.
   (F) Model for evaluating the therapeutic effect of cell condensates prepared from β cells.
   (G) Time-dependent changes in blood glucose level in diabetic model mice transplanted with cell condensates prepared from β cells.

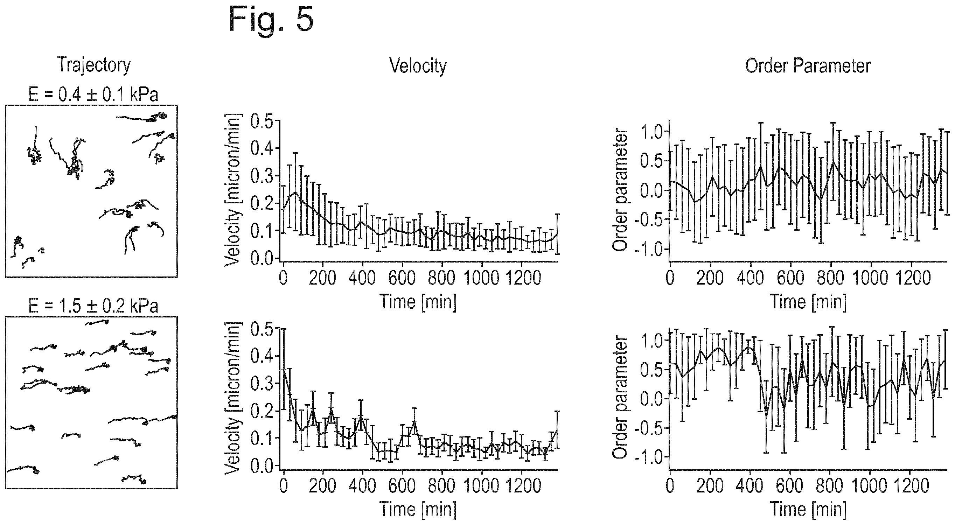

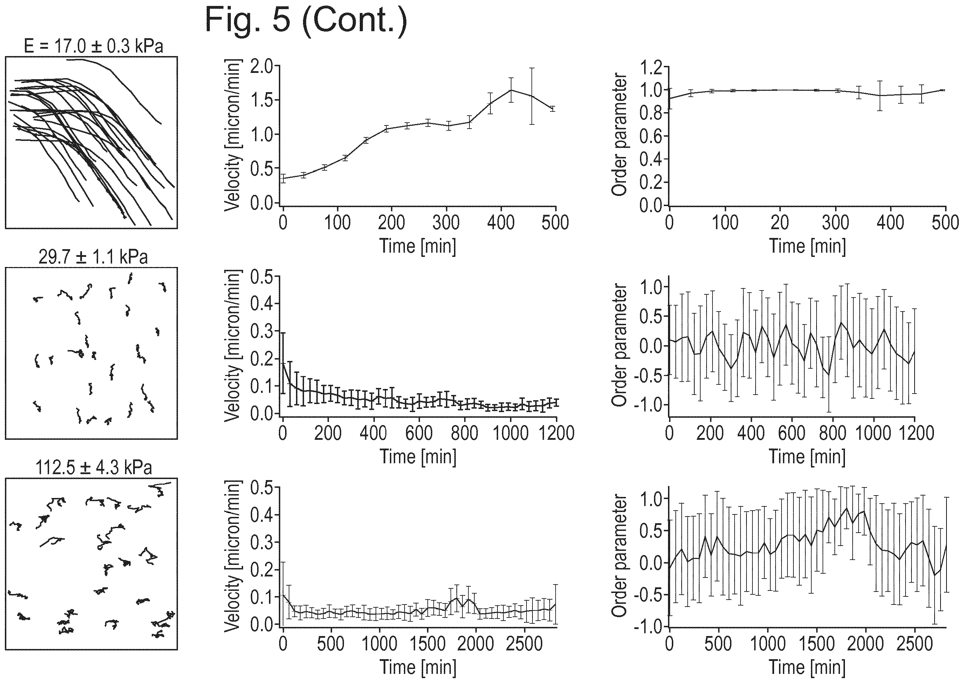

[Fig.5] Time-dependent changes in the trajectory, velocity and order parameter of MSCs in cell condensates under various stiffness conditions

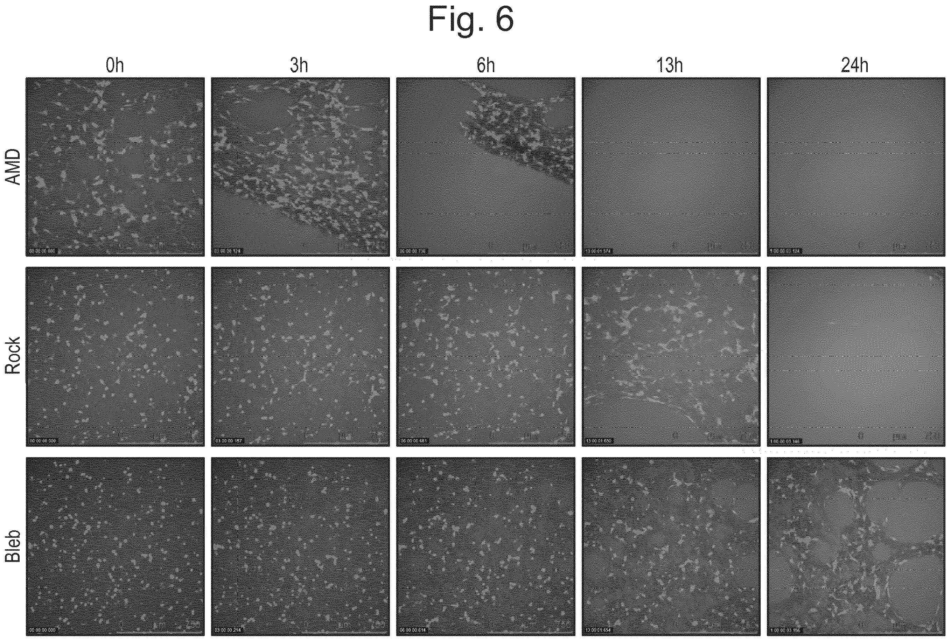

[Fig. 6] Chronological observation of cell condensate formation processes using various inhibitors.

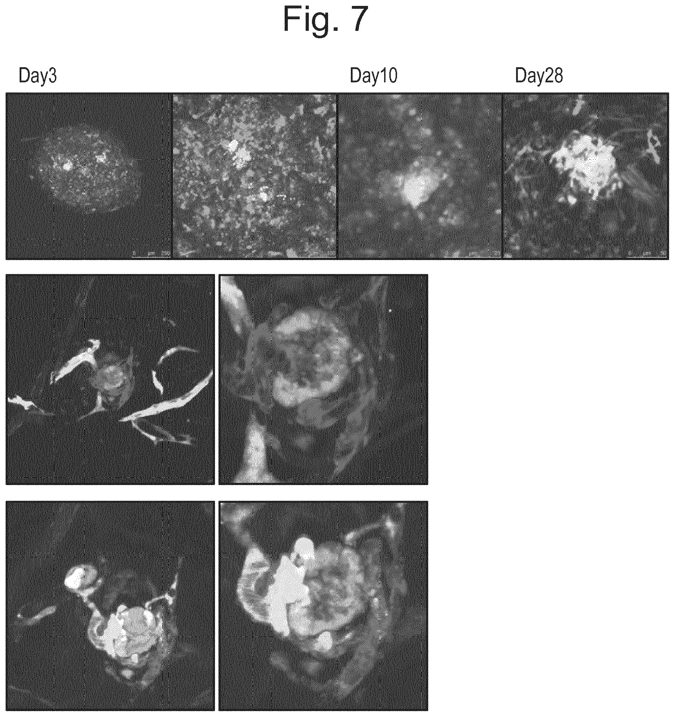

[Fig. 7] *In vivo* self-organization of cell condensate using adult kidney tissue.

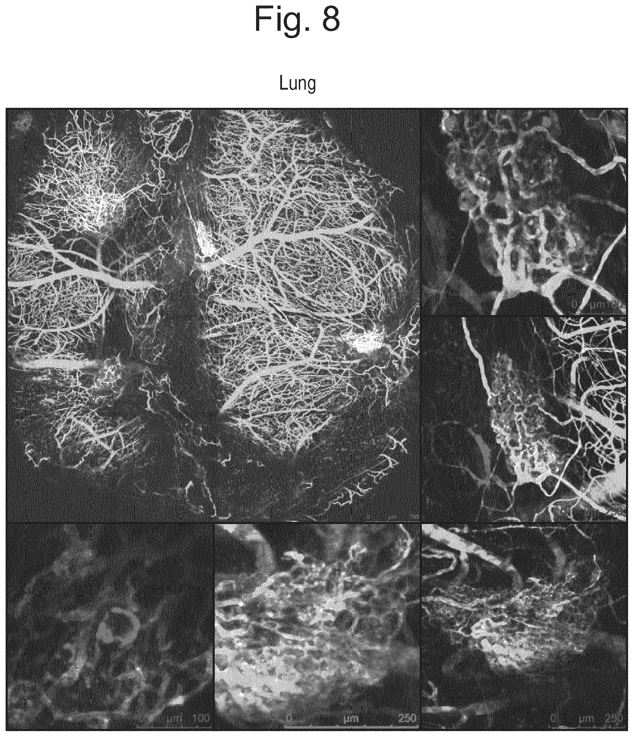

[Fig. 8] *In vivo* self-organization of cell condensate using embryonic lung tissue.

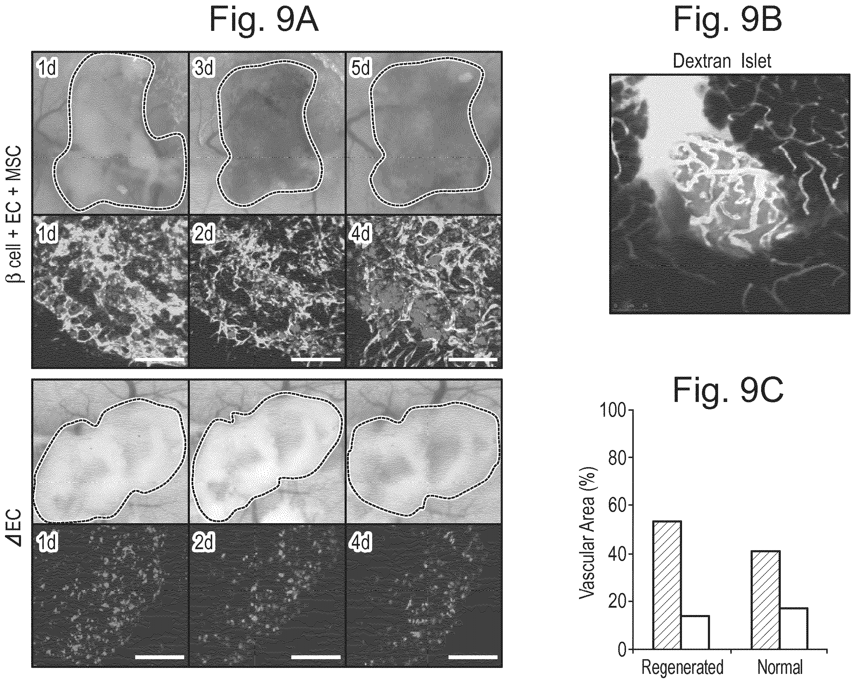

[Fig. 9] Tracing of *in vivo* vascularization process in cell condensate using β cells.

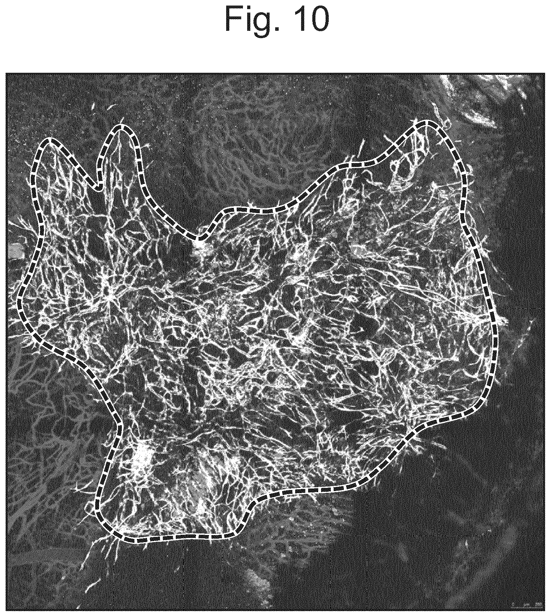

[Fig. 10] Observation of *in vivo* junctions with host blood vessels in cell condensate using β cells.

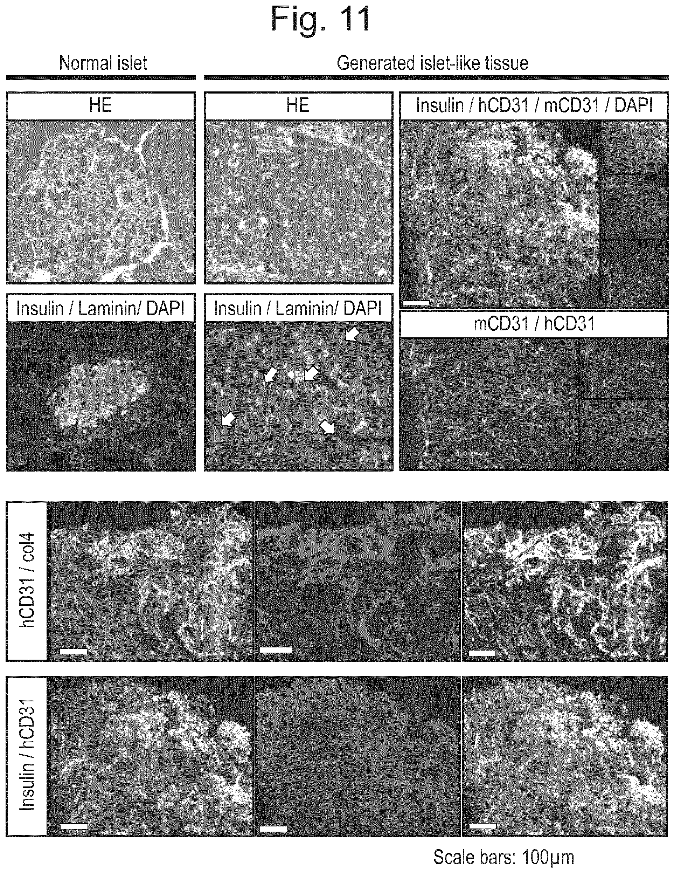

[Fig. 11] Histological analysis of tissues generated from cell condensate using β cells.

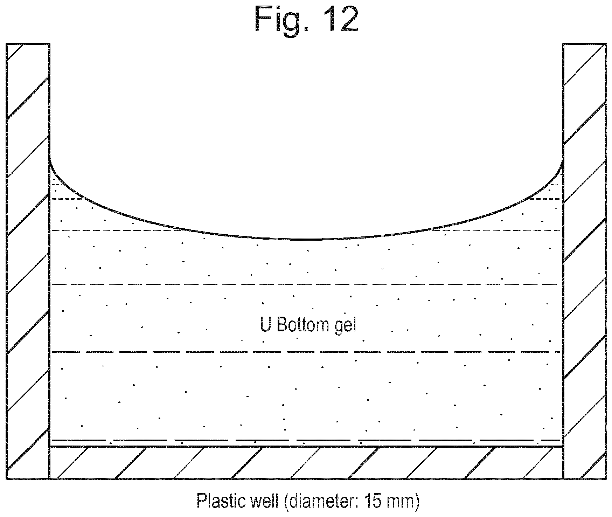

[Fig. 12] Cross section of U-bottom gel.

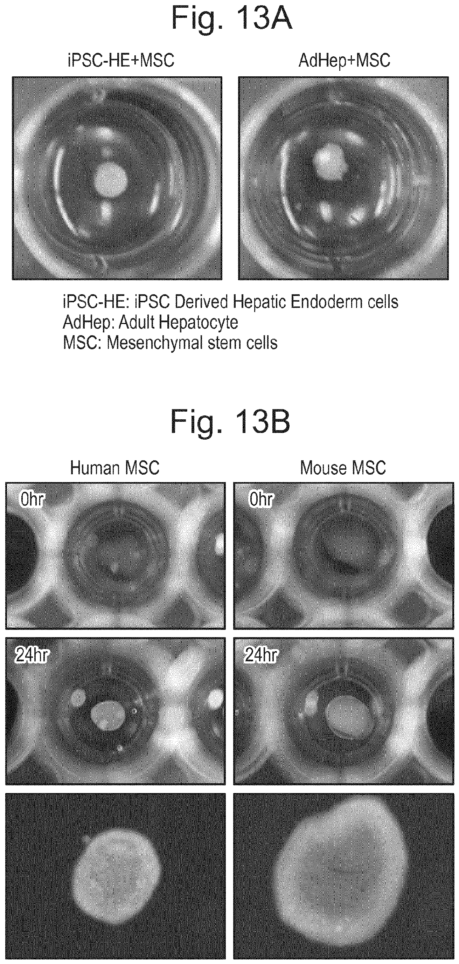

[Fig. 13] (A) Formation of cell condensates containing no vascular endothelial cells. (B) Formation of cell condensates using human or mouse mesenchymal cells.

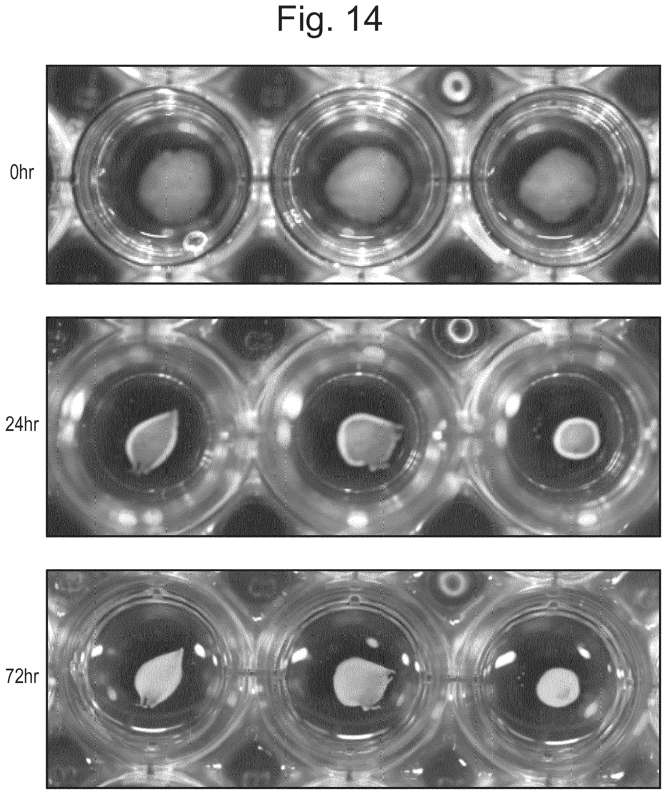

[Fig. 14] Formation of cell condensates using U-bottom gel.

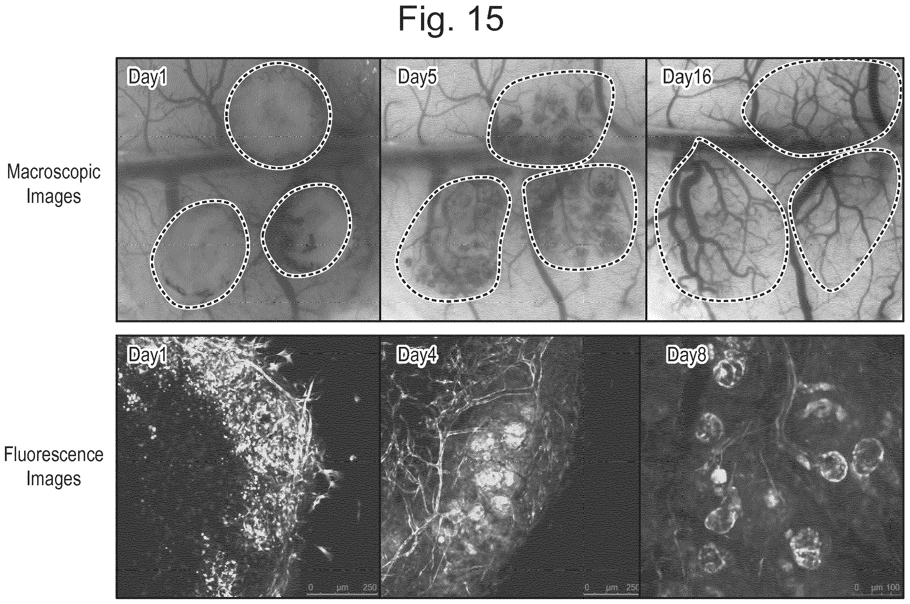

[Fig. 15] Reconstitution of a functional vascular network by transplantation of a kidney primordium prepared on a support.

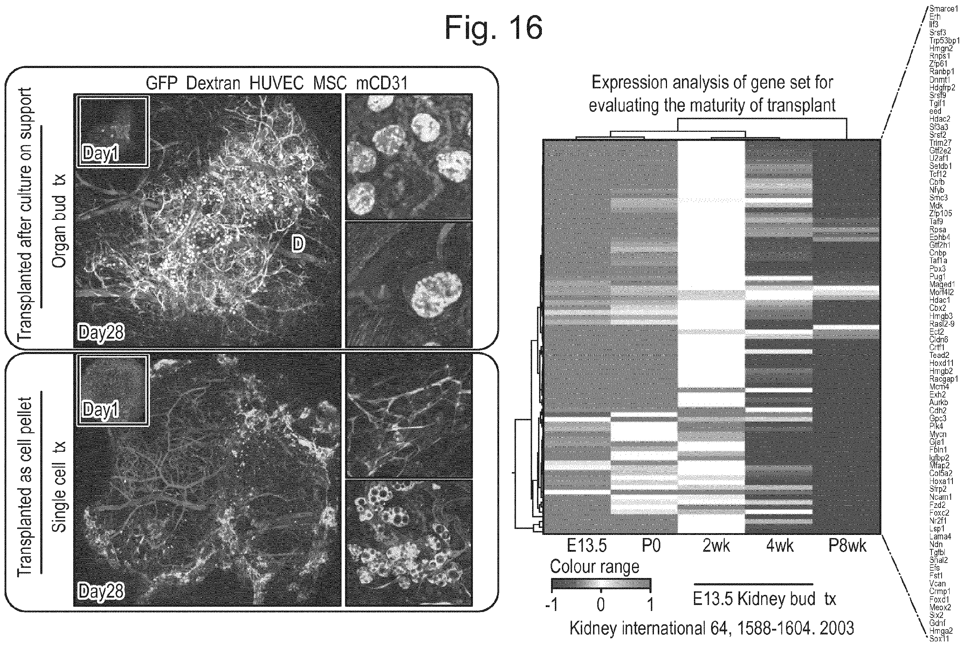

[Fig. 16] Maturation of transplanted kidney primordium.

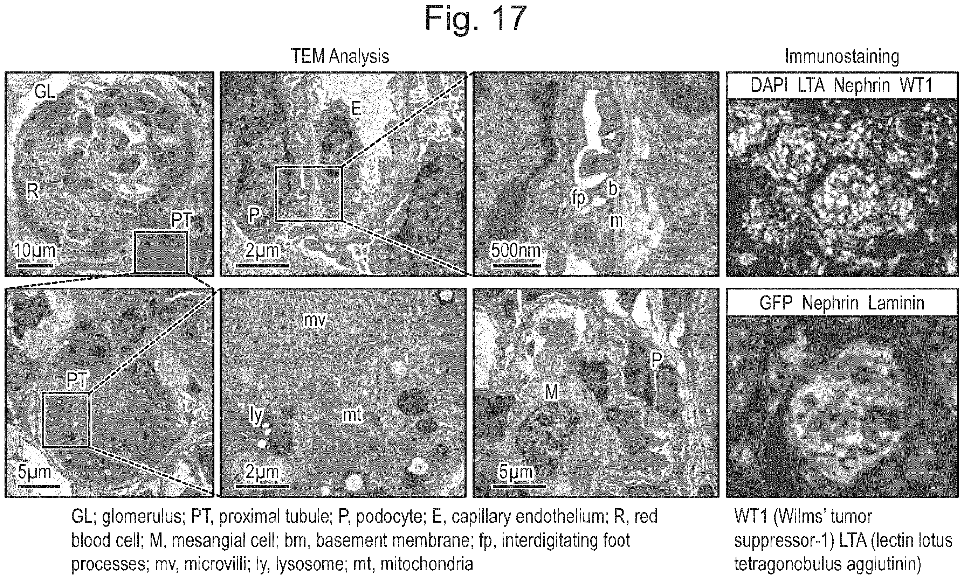

[Fig. 17] Structural analysis of kidney primordium that matured after transplantation.

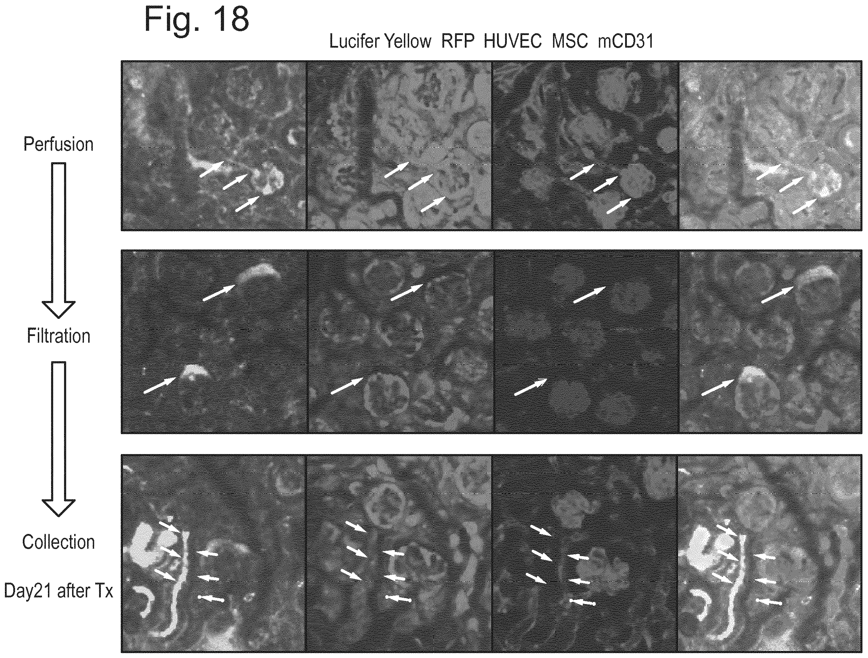

[Fig. 18] Live imaging of the capacity of the transplanted kidney primordium to produce primitive urine.

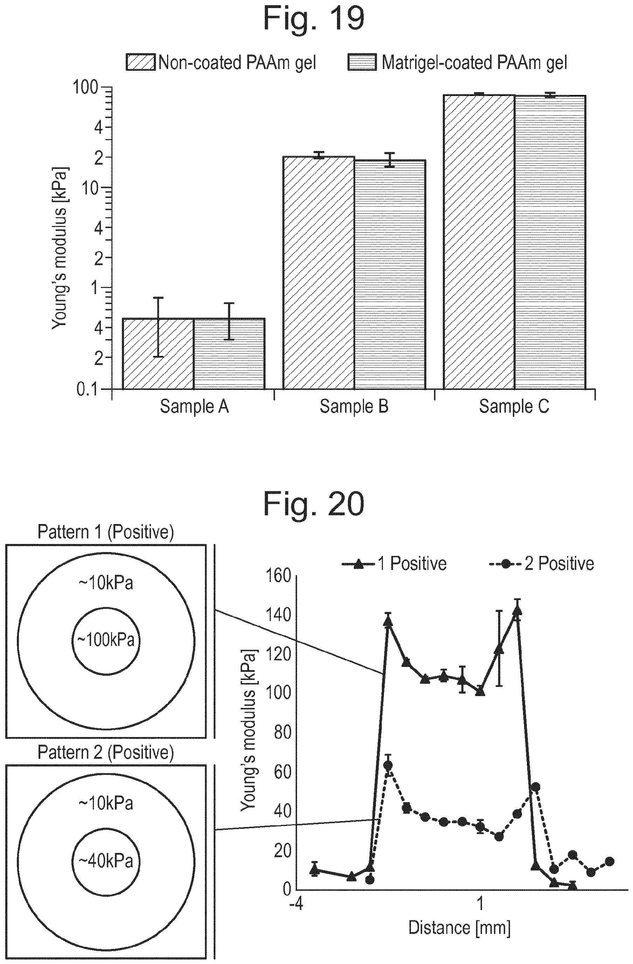

[Fig. 19] Measurements of stiffness properties before and after coating with a biochemical substance.
[Fig. 20] Preparation of supports having multiple patterns of stiffness.

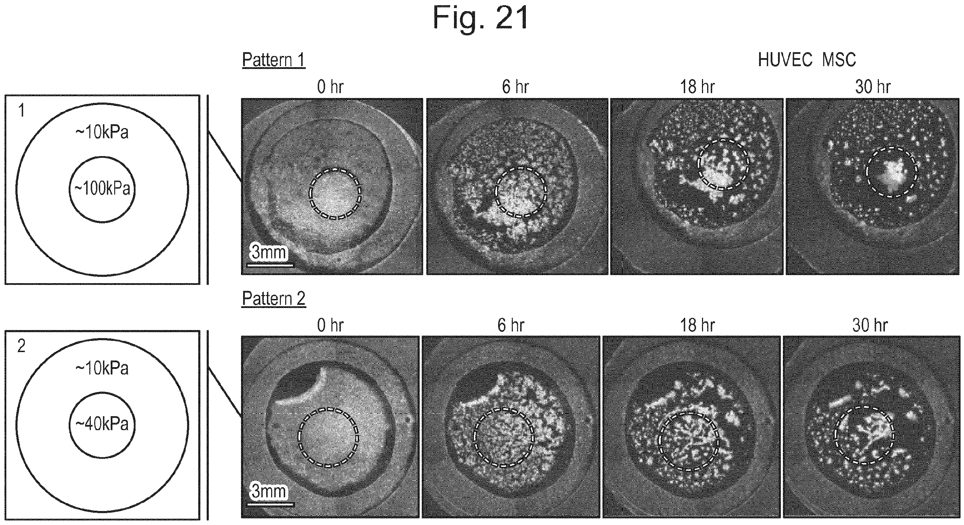

[Fig. 21] Preparation of cell condensates on supports having multiple patterns of stiffness.

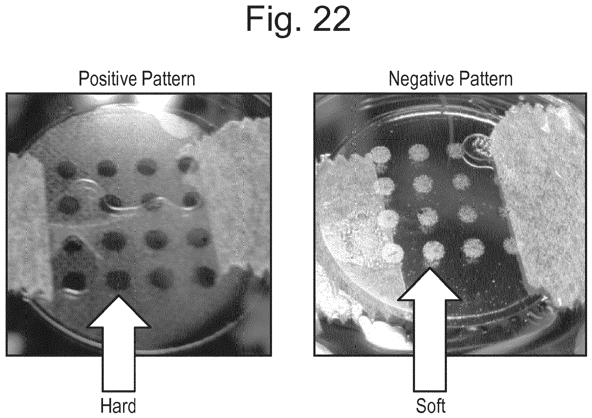

[Fig. 22] Preparation of supports having complex multiple patterns.

### BEST MODES FOR CARRYING OUT THE INVENTION

The present invention provides a method of preparing a cell condensate *in vitro,* comprising culturing a mixture of cells and/or tissues of a desired type and undifferentiated mesenchymal cells to form a cell condensate, wherein the mixture of cells and/or tissues of a desired type and undifferentiated mesenchymal cells is cultured on a gel-like support on which the undifferentiated mesenchymal cells are capable of contraction, wherein the gel-like support has a stiffness of a Young's modulus of 1-200 kPa, and wherein the cell condensate is formed autonomously.

Mesenchymal cells are connective tissue cells that are mainly located in mesoderm-derived connective tissues and which form support structures for cells that function in tissues. In the present specification, the term "mesenchymal cell" is a concept that encompasses those cells which are destined to, but are yet to, differentiate into mesenchymal cells. Mesenchymal cells to be used in the present invention are undifferentiated. Whether a cell is an undifferentiated mesenchymal cell or not can be determined by checking to see if the cell expresses marker proteins such as Stro-1, CD29, CD44, CD73, CD90, CD105, CD133, CD271 or Nestin (if any one or more of the above-listed marker proteins are expressed, the cell can safely be regarded as an undifferentiated mesenchymal cell). A mesenchymal cell in which none of the above-listed markers is expressed can be regarded as a differentiated mesenchymal cell. Among the terms used by those skilled in the art, the following are included in the "mesenchymal cell": mesenchymal stem cells, mesenchymal progenitor cells, mesenchymal cells (R. Peters et al. PLoS One. 30; 5(12):e15689 (2010)) and so on. As mesenchymal cells, human-derived ones are mainly used. However, mesenchymal cells derived from non-human animals (e.g., animals used, for example, as experimental animals, pet animals, working animals, race horses or fighting dogs; more specifically, mouse, rat, rabbit, pig, dog, monkey, cattle, horse, sheep, chicken, shark, devilfish, ratfish, salmon, shrimp, crab or the like) may also be used.

The cells and/or tissues of a desired type to be mixed with undifferentiated mesenchymal cells are independent of the types or numbers to be combined and may be any cells and/or tissues. Moreover, the origin of such cells and/or tissues also does not matter and they may be derived from any organ (e.g. liver, pancreas, intestine, lung, kidney, heart and brain) or any tissue; alternatively, they may be derived from cancer. Cells to be mixed with undifferentiated mesenchymal cells may be functional cells which constitute organs or tissues, or undifferentiated or pluripotent cells which will differentiate into functional cells. Further, tissues to be mixed with undifferentiated mesenchymal cells may be tissues isolated from individuals, or tissues induced from functional cells which constitute organs or tissues, or tissues induced from undifferentiated or pluripotent cells which will differentiate into functional cells.

Undifferentiated cells may be cells capable of differentiating into an organ such as kidney, heart, lung, spleen, esophagus, stomach, thyroid, parathyroid, thymus, gonad, brain or spinal cord; cells capable of differentiating into an ectodermal organ such as brain, spinal cord, adrenal medulla, epidermis, hair/nail/dermal gland, sensory organ, peripheral nerve or lens; cells capable of differentiating into a mesodermal organ such as kidney, urinary duct, heart, blood, gonad, adrenal cortex, muscle, skeleton, dermis, connective tissue or mesothelium; and cells capable of differentiating into an endodermal organ such as liver, pancreas, intestine, lung, thyroid, parathyroid or urinary tract. Whether or not a cell is capable of differentiating into an ectodermal organ, mesodermal organ or endodermal organ can be determined by checking for the expression of marker proteins (if any one or a plurality of marker proteins are expressed, the cell can be regarded as a cell capable of differentiating into an endodermal organ). For example, cells capable of differentiating into liver have such markers as HHEX, SOX2, HNF4A, AFP and ALB; cells capable of differentiating into pancreas have such markers as PDX1, SOX17 and SOX9; cells capable of differentiating into intestine have such markers as CDX2 and SOX9; cells capable of differentiating into kidney have such markers as SIX2 and SALL1; cells capable of differentiating into heart have such markers as NKX2-5, MYH6, ACTN2, MYL7 and HPPA; cells capable of differentiating into blood have such markers as C-KIT, SCA1, TER119 and HOXB4; and cells capable of differentiating into brain or spinal cord have such markers as HNK1, AP2 and NESTIN. Among the terms used by those skilled in the art, the following are included in the "undifferentiated cell" of the present invention: hepatoblast, hepatic progenitor cells, pancreatoblast, hepatic precursor cells, pancreatic progenitors, pancreatic progenitor cells, pancreatic precursor cells, endocrine precursors, intestinal progenitor cells, intestinal precursor cells, intermediate mesoderm, metanephric mesenchymal precursor cells, multipotent nephron progenitor, renal progenitor cells, cardiac mesoderm, cardiovascular progenitor cells, cardiac progenitor cells (JR. Spence et al. Nature.; 470(7332): 105-9.(2011); Self et al. EMBO J.; 25(21): 5214-5228.(2006); J. Zhang et al. Circulation Research.; 104: e30-e41(2009); G. Lee et al. Nature Biotechnology 25, 1468 - 1475 (2007)) and so on. Examples of pluripotent cells include pluripotent cells obtained from living bodies (e.g., ES cells), pluripotent cells obtained by induction from reprogramming [e.g., iPS cells, STAP cells (Stimulus-triggered fate conversion of somatic cells into pluripotency. Nature, 2014), MUSE cells (Multilineage-differentiating stress-enduring (Muse) cells are a primary source of induced pluripotent stem cells in human fibroblasts. PNAS, 2011), iMPC cells (induced multipotent progenitor cell; Mouse liver repopulation with hepatocytes generated from human fibroblasts. Nature, 2014)] and combinations thereof. Undifferentiated cells may be prepared from pluripotent stem cells such as induced pluripotent stem cells (iPS cells) or embryonic stem cells (ES cells) according to known methods. For example, cells capable of differentiating into liver may be prepared as previously described (K.Si-Taiyeb et al. Hepatology, 51 (1): 297- 305(2010); T. Touboul et al. Hepatology. 51 (5): 1754-65 (2010)); cells capable of differentiating into pancreas may be prepared as previously described (D. Zhang et al. Cell Res.;19(4):429-38 (2009)); cells capable of differentiating into intestine may be prepared as previously described (J. Cai et al. J Mol Cell Biol.; 2(1):50-60 (2010); R. Spence et al. Nature.; 470 (7332):105-9 (2011)); cells capable of differentiating into heart may be prepared as previously described (J. Zhang et al. Circulation Research.; 104: e30-e41(2009); and cells capable of differentiating into brain or spinal cord may be prepared as previously described (G. Lee et al. Nature Biotechnology 25, 1468 - 1475 (2007)). Examples of functional cells that constitute organs or tissues include endocrine cells in pancreas, pancreatic duct epithelial cells in pancreas, hepatocytes in liver, epithelial cells in intestine, tubular epithelial cells in kidney, glomerular epithelial cells in kidney, cardiomyocytes in heart, lymphocytes, granulocytes and erythrocytes in blood, neurons and glial cells in brain, as well as neurons and Schwan cells in spiral cord. Human-derived cells are mainly used, but cells derived from non-human animals (e.g., animals used, for example, as experimental animals, pet animals, working animals, race horses or fighting dogs; more specifically, mouse, rat, rabbit, pig, dog, monkey, cattle, horse, sheep, chicken, shark, devilfish, ratfish, salmon, shrimp, crab or the like) may also be used.

When a cell condensate need be provided with a vascular system, vascular cells may be added to a mixture of cells and/or tissues of a desired type with undifferentiated mesenchymal cells. Vascular cells may be isolated from vascular tissues but they are in no way limited to those isolated from vascular tissues. Vascular cells may be derived from pluripotent cells (such as iPS cells and ES cells) or from non-human totipotent cells by directed differentiation. As vascular cells, vascular endothelial cells are preferable. In the present specification, the term "vascular endothelial cells" means cells that constitute vascular endothelium or cells that are capable of differentiating into such cells (for example, vascular endothelial progenitor cells and vascular endothelial stem cells). Whether a cell is a vascular endothelial cell or not can be determined by checking to see if it expresses marker proteins such as TIE2, VEGFR-1, VEGFR-2, VEGFR-3, VE-cadherin and CD31 (if any one or more of the above-listed marker proteins are expressed, the cell can safely be regarded as a vascular endothelial cell). Further, as markers for vascular endothelial progenitor cells, c-kit, Sca-1, etc. have been reported. If these markers are expressed, the cell of interest can be identified as a vascular endothelial progenitor cell (S. Fang et al., PLOS Biology, 2012; 10(10): e1001407). Among the terms used by those skilled in the art, the following are included in the "vascular endothelial cell" of the present invention: endothelial cells, umbilical vein endothelial cells, endothelial progenitor cells, endothelial precursor cells, vasculogenic progenitors, hemangioblast (HJ. Joo et al. Blood. 25; 118(8): 2094-104 (2011)) and so on. As vascular cells, human-derived cells are mainly used. However, vascular cells derived from non-human animals (e.g., animals used, for example, as experimental animals, pet animals, working animals, race horses or fighting dogs; more specifically, mouse, rat, rabbit, pig, dog, monkey, cattle, horse, sheep, chicken, shark, devilfish, ratfish, salmon, shrimp, crab or the like) may also be used. Vascular cells may be obtained from umbilical cord blood, umbilical cord vessels, neonatal tissues, liver, aorta, brain, bone marrow, adipose tissues, and so forth.

In the present specification, the term "vascular system" refers to a structure composed of vascular endothelial cells and their supporting cells. Vascular systems not only maintain tissues but also play an important role in the maturation process of tissues. Vascular structures have such a role that, once transplanted, they supply the interior of tissues with oxygen and nutrients that are necessary for their survival. What is more, it is believed that even before blood flows into the tissue, recapitulating three-dimensional tissue structures and cell polarities that are accompanied by blood vessels is important for the differentiation, proliferation and maintenance of cells. Therefore, avascular tissues not only fail to engraft upon transplantation, resulting in necrosis of their interior, but at the same time, tissue maturation associated with vascularization is not achieved. It has, therefore, been difficult for avascular tissues to exhibit adequate functions.

In the present specification, the terms "providing a vasculature system" and "vascularization" mean that a vascular system composed of vascular endothelial cells and their supporting cells is made directly integral with a target tissue. When a biological tissue that has been provided with a vascular system is transplanted into a living body, maturation of blood vessels is observed and upon connecting to the host blood vessels, blood perfusion starts, enabling the transplanted biological tissue to be directed to a functional tissue/organ having vascular networks.

In the present invention, a mixture of cells and/or tissues of a desired type (in a total cell count of 400,000 or more, preferably 400,000 to 4,400,000, and more preferably about 2,000,000) and undifferentiated mesenchymal cells (40,000 or more, preferably 50,000 to 1,000,000, and more preferably 100,000 to 400,000 cells) may be cultured. According to the method of the present invention, a cell condensate is formed autonomously. Cell condensates of various sizes can be formed, e.g., in sizes of 1 mm or more (preferably 1-20 mm and more preferably 1-8 mm). The ratio between the cells and/or tissues of a desired type and the undifferentiated mesenchymal cells is not particularly limited as long as it falls within a range which permits formation of cell condensates of a desired size. An advantageous cell count ratio between the cells and/or tissues of a desired type and the mesenchymal cells is 10 : 0.5-3.

When vascular cells are added, 4,000 or more (preferably 20,000 to 400,000, more preferably about 40,000 to 280,000) vascular cells may be added to cells and/or tissues of a desired type (in a total cell count of 400,000 or more, preferably 400,000 to 4,400,000, and more preferably about 2,000,000) and undifferentiated mesenchymal cells (40,000 or more, preferably 50,000 to 1,000,000, and more preferably 100,000 to 400,000 cells). The ratio between the cells and/or tissues of a desired type, undifferentiated mesenchymal cells and vascular cells is not particularly limited as long as it falls within a range which permits formation of cell condensates of a desired size. An advantageous cell count ratio between the cells and/or tissues of a desired type, undifferentiated mesenchymal cells and vascular cells is 10 : 1-3 : 0.1-7.

The mixture of the cells and/or tissues of a desired type and the undifferentiated mesenchymal cells is capable of forming cell condensates in two-dimensional culture. The medium used for culture may be any medium that enables the formation of cell condensates. Preferably, the medium has a composition that promotes induction of self-organization into a tissue of interest. For example, when self-organization is to be induced by transplantation into a living body, a medium prepared by mixing a vascular endothelial cell culture medium and a medium for culturing the organ of interest at 1:1 may be used. Preferable examples of vascular endothelial cell culture media include, but are not limited to, EGM^{™} BulletKit^{™} (Lonza CC-4133) and EGM-2^{™}, BulletKit (Lonza CC-3162), EGM-2^{™} and MV (Lonza CC-3156). Examples of media for culturing organs include, but are not limited to, RPMI1640 (Wako) supplemented with 20% fetal bovine serum (BWT Lot. S-1560), 100 µg/ml penicillin/streptomycin (Gibco) and Insulin-Transferrin-Selenium X (GIBCO), which may be used for adult renal cells. For culturing embryonic renal cells, D-MEM High-Glucose (Wako 043-30085), 10% fetal bovine serum (BWT Lot.S-1560), 100 µg/ml penicillin/streptomycin (Gibco), and the like may be preferably used.

The mixture of the cells and/or tissues of a desired type and the undifferentiated mesenchymal cells may be cultured on a gel-like support on which the undifferentiated mesenchymal cells are capable of contraction.

Contraction of undifferentiated mesenchymal cells may be confirmed, for example, by microscopically or macroscopically noting the formation of a 3D tissue morphologically or by showing that the tissue has such a strength that it retain its shape as it is collected as with a spatula (Takebe et al. Nature 499 (7459), 481-484, 2013).

The support may be a gel-like substrate having an appropriate stiffness [e.g., a Young's modulus of 200 kPa or less (in the case of a Matrigel-coated gel of a flat shape); however, the appropriate stiffness of the support may vary depending on the coating and shape]. Examples of such substrates include, but are not limited to, hydrogels (such as acrylamide gel, gelatin and Matrigel). The stiffness of the support need not be uniform and may vary depending on the shape, size and quantity of a condensate of interest. It is possible to provide the stiffness with a spatial/temporal gradient (as in Example 6 to be described later) or a pattern (as in Example 7 to be described later). When the stiffness of the support is uniform, it is preferably 100 kPa or less, more preferably 1-50 kPa. The gel-like support may be planar, or the side on which culture is to be performed may have a U- or V-shaped cross section. If the side of the gel-like support on which culture is to be performed has a U- or V-shaped cross section, cells tend to gather on the culture surface and a cell condensate can advantageously be formed from a smaller number of cells and/or tissues. Further, the support may be modified chemically or physically. Examples of modifying substances include, but are not limited to, Matrigel, laminin, entactin, collagen, fibronectin and vitronectin.

One example of the gel-like culture support that is provided with a spatial gradient of stiffness is a gel-like culture support whose stiffness in the central part is greater than the stiffness in the peripheral part (see Example 6 to be described later and Figs. 20 and 21). Appropriately, the stiffness of the central part is 200 kPa or less and it suffices that the peripheral part is softer than the central part. Appropriate values for the stiffness of the central and peripheral parts of the substrate are variable depending on the coating and the shape. Another example of the gel-like culture support that is provided with a spatial gradient of stiffness is a gel-like culture support whose stiffness in the peripheral part is greater than the stiffness in the central part.

One example of the patterned, gel-like culture support is a gel-like culture support having one or more patterns in which the stiffness of the central part is greater than the stiffness of the peripheral part (see Example 7 to be described later; Fig. 22, left panel: positive pattern). Appropriately, the stiffness of the central part is 200 kPa or less; it suffices that the peripheral part is softer than the central part. Appropriate values for the stiffness of the central and peripheral parts of the substrate are variable depending on the coating and the shape. Another example of the patterned, gel-like culture support is a gel-like culture support having one or more patterns in which the stiffness of the peripheral part is greater than the stiffness of the central part (see Example 7 to be described later; Fig. 22, right panel: negative pattern). Appropriately, the stiffness of the peripheral part is 200 kPa or less; it suffices that the central part is softer than the peripheral part. Appropriate values for the stiffness of the central and peripheral parts of the substrate are variable depending on the coating and the shape.

The temperature at the time of culture is not particularly limited but it is preferably 30-40°C and more preferably 37°C. When a larger tissue is to be cultured, an increased amount of oxygen is preferably supplied into the incubator. The amount of oxygen supply is appropriately 4-50%, preferably 10-30%, and more preferably 18-25%.

The culture period is not particularly limited but it is preferably 12-144 hr. For example, when formation of cell condensates 0.4-10 mm in size from cells and/or tissues isolated from liver is intended, the culture period is preferably 12-48 hr. When formation of cell condensates 0.4-10 mm in size from cells and/or tissues isolated from pancreas is intended, the culture period is preferably 12-144 hr. When formation of cell condensates 0.4-3 mm in size from cells and/or tissues isolated from intestine is intended, the culture period is preferably 12-96 hr. When formation of cell condensates 0.4-1 mm in size from cells and/or tissues isolated from lung is intended, the culture period is preferably 12-96 hr. When formation of cell condensates 0.4-10 mm in size from cells and/or tissues isolated from heart is intended, the culture period is preferably 12-96 hr. When formation of cell condensates 0.4-5 mm in size from cells and/or tissues isolated from kidney is intended, the culture period is preferably 12-144 hr. When formation of cell condensates 0.4-10 mm in size from cells and/or tissues isolated from brain is intended, the culture period is preferably 12-144 hr. When formation of cell condensates 0.4-10 mm in size from cells and/or tissues isolated from cancer is intended, the culture period is preferably 12-144 hr. Further, when formation of cell condensates 0.4-10 mm in size from pluripotent cells such as iPS cells is intended, the culture period is preferably 48-144 hr.

In the cell condensates prepared by the method of the present invention, cell-cell interactions have taken place in such a close manner that a biological environment as occurs in the womb is recapitulated. As a consequence, induction of early differentiation into organ progenitor cells occurs efficiently and this would improve the frequency and number of such cells. Further, in the cell condensates prepared by the method of the present invention, cells adhere to each other so strongly that they can be collected in a non-destructive manner.

The cell condensate described herein is a concept typically encompassing organ buds and organoids [organ bud (WO2013/047639), liver bud, liver diverticula, liver organoid, pancreatic (dorsal or ventral) buds, pancreatic diverticula, pancreatic organoid, intestinal bud, intestinal diverticula, intestinal organoid (K. Matsumoto et al. Science. 19; 294 (5542): 559-63 (2001)]. The cell condensates are independent of the types of constituent cells and the number of such types. However, organ buds correspond to cell condensates that are formed at an early stage of organogenesis and are in principle composed of the following three types of cells: functional cells that constitute organs or tissues (or undifferentiated cells which will differentiate into functional cells); vascular cells; and mesenchymal cells. Organoids are solely composed of cells that constitute epithelial tissues and they are basically of a small size (1 mm or less).

Cell condensates undergo self-organization to form three-dimensional tissue structures provided with higher structures, whereby progenitor cells can be directed to terminal differentiation. Self-organization may be performed either *in vivo* or *in vitro.* For example, when a cell condensate prepared by the method of the present invention is transplanted into a living body, vascular networks are formed, blood perfusion is induced, and self-organization into a higher tissue with a complex structure occurs, enabling the preparation of tissues/organs that have a highly ordered tissue structure comparable to that of adult tissues. With the cell condensate described herein, it may be possible to prepare a higher tissue that is provided with not only a vascular network but also higher structures such as ureteral structure, biliary structure, tracheal structure, etc. Further, a great number of organs essentially require that reconstitution associated with other organs be realized in order to exhibit their functions; e.g., in liver, reconstitution of junctions with bile duct and pancreatic duct and connection to duodenum is essential for exhibiting its function. According to the present invention, a cell condensate which recapitulates interactions with other organs is prepared. This cell condensate is expected to find use as a system for inducing self-organization into complex organs existing in the body.

Further, the present invention also provides a method of preparing a three-dimensional tissue structure, comprising preparing a cell condensate by the above-described method and allowing self-organization of the cell condensate.

### EXAMPLES

### [Example 1]

It has been long held that formation of cell aggregation is an important principle for isolated immature cells to form a three-dimensional, complex organ via self-organization. The present inventors had found that liver primordia (of millimeter scale) were autonomously formed from isolated human liver progenitor cells *in vitro* by recapitulating the cell-cell interactions which would occur at organogenesis stages. However, the mechanism underlying such dynamic three-dimensional organization were totally unknown. The present inventors revealed that this 3D tissue formation started from self-assembly behavior of multiple cell units and that the presence of the cytoskeletal contractile force of myosin II occurring in mesenchymal stem cells was crucial for the progress of such behavior. This dynamic cell collective behavior is regulated by the stiffness conditions of substrate matrix. Further, the present inventors succeeded under optimized substrate conditions in preparing three-dimensional organ primordia from cells/tissues isolated from diverse organs including liver, pancreas, intestine, lung, heart, kidney, brain and even cancer. The thus prepared three-dimensional primordia were immediately vascularized upon transplantation (since vascular endothelial cells had been incorporated therein), followed by autonomous formation of self-organized three-dimensional tissue structures having therapeutic effects. Toward the goal of regenerative medicine in future, this principle will serve to establish a highly versatile platform for reconstituting a plurality of vascularized, complex organ systems from stem cells via dynamic cell condensation and the subsequent self-organization.

It is known that liver is formed from a condensed tissue mass termed "liver bud" at week 5-8 of gestation in human during physiological organogenesis. Cell-cell interactions between mesenchymal stem cells, undifferentiated vascular endothelial cells and anterior visceral endoderm cells are required for the initiation of liver regeneration termed "liver budding" (also called "liver bud") in the foregut (1). In parallel with these basic understandings in organogenesis, recent advances in regenerative medicine have also demonstrated that this dynamic three-dimensional (3-D) rearrangement can be mimicked by recapitulating cellular interactions at organogenesis stages in culture using pluripotent stem cells (PSCs). When plated on a solidified soft matrix gel, single PSC-derived hepatocytes autonomously form 3-D condensates by co-culture with endothelial cells and mesenchymal cells (2). Once condensates are established, they continue to self-organize after several days under complete *in vitro* conditions into liver bud tissues having a structure resembling the organs that exist in the womb (3). The *in vitro* grown organ bud is transplanted into a living body, where it undergoes further self-organization (is matured) to eventually become a vascularized and functional liver. This method opens a new road for artificial reconstitution of vascularized organ systems (4). The most attractive aspect of these previous observations was that, in spite of the culture on a flat two-dimensional culture plate, considerably great morphogenetic changes were found in the cocultured cells. In the preceding studies of self-organization, condensates of micron scale were generally produced in 96-well plates with steep bottoms. In the system under consideration, however, condensates are capable of growing up to millimeter or even centimeter scale (5, 6). It was therefore the principal object of the present study to analyze the mechanism working at the center of this surprisingly dynamic assembling behavior and to elucidate crucial factors for recapitulating the phenomenon of interest. And under optimized conditions, the present inventors assessed the expandability of this approach ultimately aiming to reconstitute other organ systems.

First, time-lapse imaging analysis was carried out to track cellular movements during organoid formation. Hepatic endoderm cells derived from human induced pluripotent stem cells (iPSCs), umbilical cord-derived endothelial cells (HUVECs), and mesenchymal stem cells (MSCs) were labeled with distinctive fluorescent markers and cocultured on a solidified matrix gel which was already described. Live cell tracking revealed that after rapid cell convergence, the assembly of vascularized organoids was initiated; this was followed by spatial rearrangements via self-organization as demonstrated by the formation of an endothelial-like network (Fig. 1). Briefly, during the initial self-convergent phase, it was discovered that cells behave as a cohesive multicellular unit and quickly travel to a single center (Fig. 1). To elucidate dynamics of such condensate formation in more detail, the temporal development of the position of the edge of the cell condensate (square root of cell area) and circularity were examined by image analysis (Fig. 1b). The results showed that cell condensates contracted gently at 10 µm/h or less up to about 7 hr after seeding, and then the contraction accelerated to about 500 µm/h at naxunyn over the next several hours and finally decreased exponentially to converge. On the other hand, its circularity decreased almost monotonically right after cell seeding and reached a minimal value of about 0.5 in 10-13 hr. The circularity then increased and finally achieved an almost constant value (0.85) at 20 hr after seeding.

The results described so far suggest that the formation of the condensate in the present study is based not on cell migration but on cell tissue contraction. First, the maximum velocity of the condensate edge reached as high as about 500 µm/h at 10-15 hr after cell seeding which is much higher than general cell migration velocity. Finally, the velocity decreased exponentially, but this suggests that the condensate is contracting in line with Kelvin-Voigt model, a dynamic model shown by an exponential function. Indeed, it has been shown that contraction of diverse cell tissues and stress fibers can be approximated with Kelvin-Voigt model. About 10 hr was required for the initiation of large-scale contraction of the condensate, which is assumed to be reasonable as a time for the progress of cell-cell adhesion and formation of stress fiber necessary for contraction. Indeed, the circularity results indicated that the shape of the condensates deviated from an exact circle during the early 10 hr, causing them to contract in distorted forms. This is believed to suggest that the contraction force at early stages of condensate formation is equal to or below the adhesion strength of cell-extracellular environment (cell-substrate and cell-container wall).

To identify the cell types which are critical for initiating this dynamic and directed cell condensation phenomenon, all the possible combinations of the three cell lineages were examined in coculture. As a result, it was found that lack of mesenchymal stem cells (MSCs) leads to a failure in condensate formation (iPSC+EC, EC, iPSC in Fig. 1). On the other hand, combination with MSCs is a sufficient condition for cell condensate formation, but the presence of vascular endothelial cells is not essential. For example, cell condensate formation was possible in coculture of iPSC-derived hepatic endoderm cells and MSC (iPSC+MSC) or coculture of vascular endothelial cells and MSC (EC+MSC) (Fig. 1). Although condensates were formed even in single MSC culture, culture groups without MSC simply produced sheet-like fragile tissues in any of the following groups: EC alone, iPSC alone, and iPSC+EC. Since it was impossible to collect such fragile tissues in a non-destructive manner, no condensates were formed. Condensate formation was not recognized also when cells were not cultured on a support (2-D, iPSC+EC+MSC). To elucidate the "contraction mechanisms" implied by the above-described observation, the contributions of the contraction force of MSCs at the molecular level against their substratum and the surrounding cells were subsequently assessed. During embryonic invagination in early developmental process, a group of cells undergoes contraction and it is known that the drastic inward displacement of cell-cell junctions is driven by myosin II (MII) activity, allowing cells to invaginate during embryonic gastrulation. The MII activity was therefore assessed by measuring time-course-dependent changes in MIIA phosphorylation with MIIA inactivating S1943 (pS1943) through decomposition of myofilament by phosphate-specific antibodies (7) and intracellular flow cytometry. Based on the formula reported to estimate MIIA activity (8), it was shown that active MIIA was remarkably up-regulated in stromal cells during condensate formation and reached its peak at 6 hr, which corresponds to the time at which cells moved at maximum velocity (1). On the other hand, it is seen that activated MIIA is almost constant throughout condensate formation in iPSC-derived hepatocytes. This suggests that the MSC-driven activation of MIIA is responsible for this strong three-dimensional rearrangement. As data indicating direct evidence for the decrease of this activated MIIA, it was shown that this condensate formation could be completely antagonized by treatment with blebbistatin (an MII ATPase inhibitor) (9). Similarly, it was found that addition of Rho kinase inhibitor Y-27632 to the cocultures partially delayed condensate formation (Fig. 1). On the other hand, with respect to the recently reported collective cell migration mechanism by an autonomously generated chemokine gradient during organogenesis (1), it was assumed that such mechanism is hard to apply because pharmacological inhibition of chemokine receptor pathways by addition of AMD3100 could not hinder condensate formation (10). These results revealed that the contraction force produced by actomyosin cytoskeleton plays an important role in the directed and drastic movements of cell condensates.

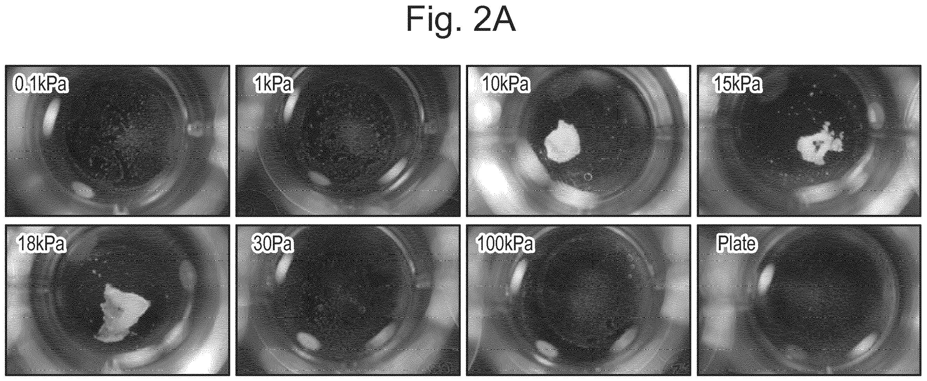

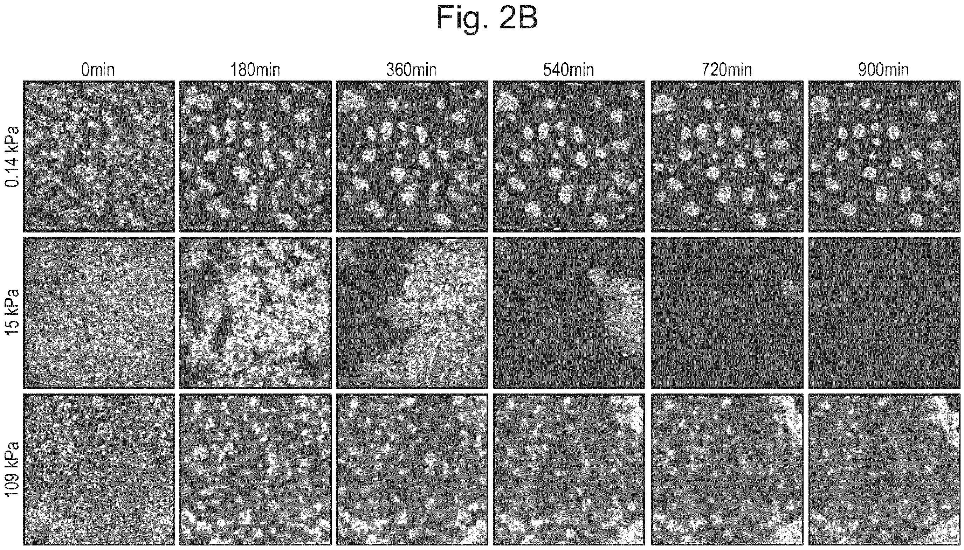

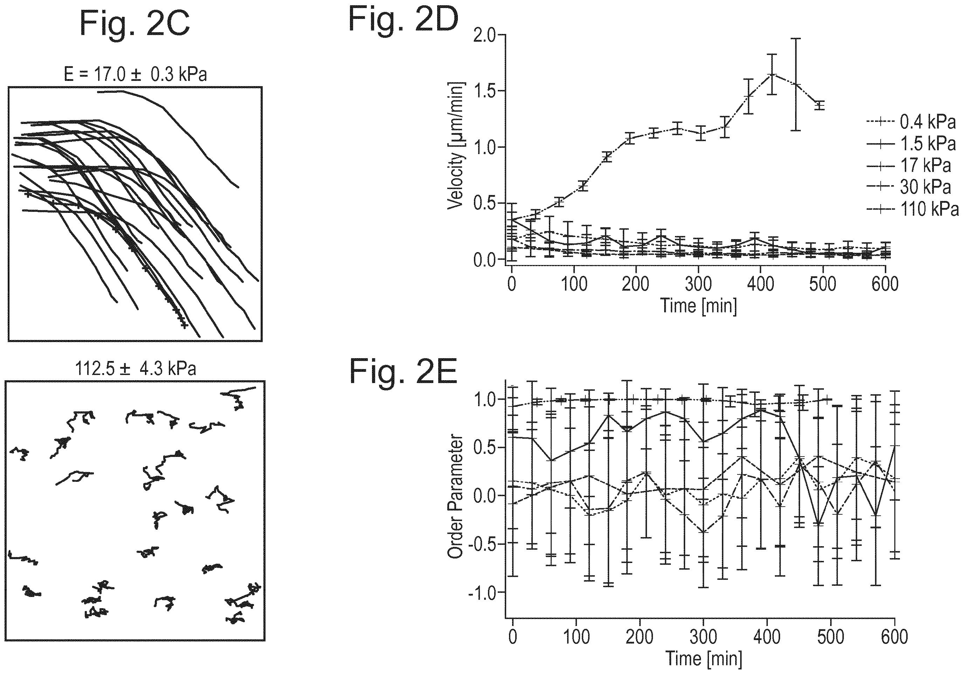

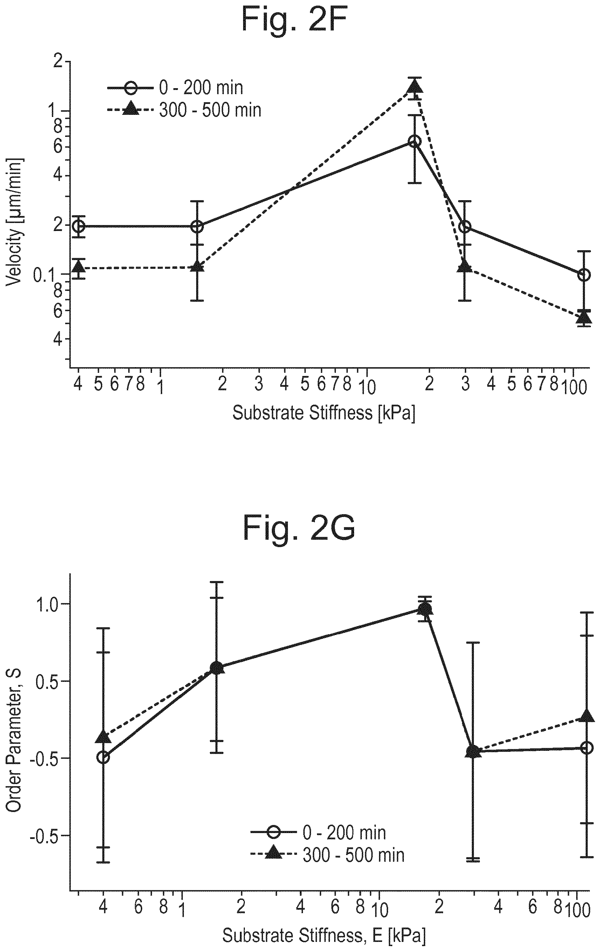

It is suggested at the single cell level that such cellular cytoskeletal contraction in culture is balanced by the degree of attachment to the anchoring matrix (11). Briefly, recent studies measuring the traction force of single cells have shown that cytoskeletal tension can be modulated by the biochemical and biophysical parameters of the substratum (12). Therefore, it was assumed that the modulation of substratum hardness conditions could alter the collective behavior of the cultured cells if this process is also applicable to the contraction mechanism in cell condensation. In preceding studies, various biochemical conditions were tested using hydrogels, collagens, laminin, entactin, and combinations thereof and showed that a basement membrane composite, such as Matrigel, is the most efficient matrix. To further clarify the essential parameters, the effect of the biophysical stiffness of substrate was assessed. Specifically, to assess the effect of the outer environment on cell response, hydrogels were prepared whose biochemical/dynamic conditions were freely tunable (Fig. 2) (13). Cells were plated on the above-prepared substrates with diverse stiffness conditions. After incubation for about 24 hr, significant differences in collective behavior were already discernible. Briefly, when the movement of MSCs during condensate formation were traced to analyze velocity and order parameter, it became clear that both the velocity and order parameters exhibited maxima at E=17 kPa. These results clearly show that the stiffness of the extracellular environment is one of the critical parameters in condensate formation. Indeed, MSCs that are the key cell in condensate formation in the system of the present invention are known to exhibit mechano-response in diverse processes including differentiation and attachment. Generally, for the formation of condensates such as spheroids, cell-cell interactions must exceed cell-extracellular interactions and this condition may have been realized in the present system by the extracellular stiffness environment of E=17 kPa. Considering the necessity of MSC (Fig. 1), it was concluded that contraction of mesenchymal cells against softer substrate might have caused these collective behaviors in coculture systems.

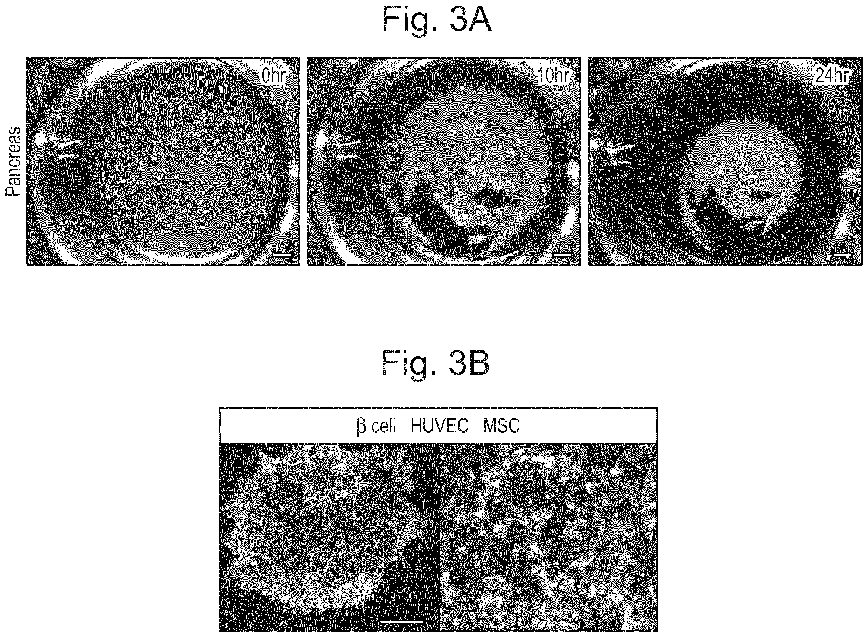

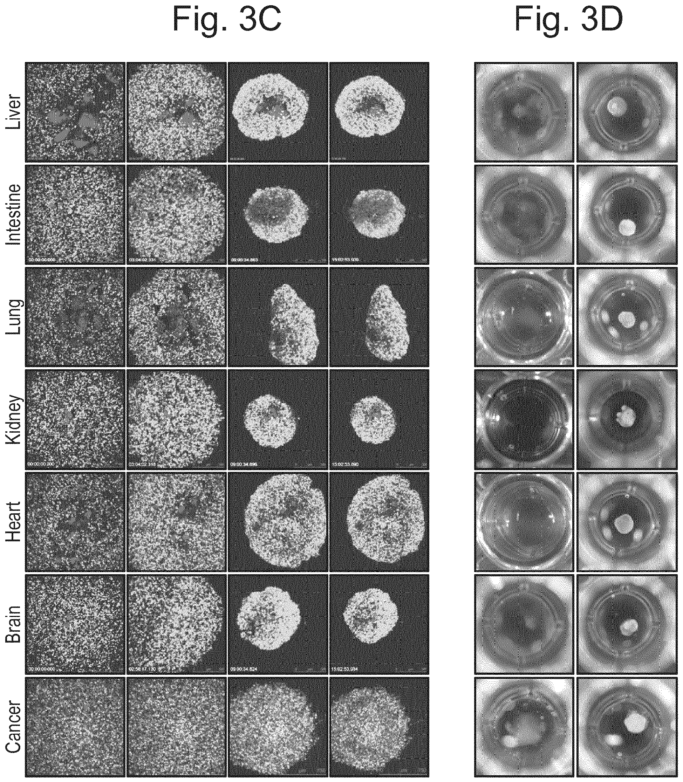

Considering that the MSC-derived contraction force plays a central role in the above-described self-assembly behavior, it may be assumed that the proposed principle can be expanded to self-organization systems for other organs irrespective of the origin of germ layers that are to be used in the future for the purpose of regenerative medicine. To validate this hypothesis, pancreatic cells were first selected and subjected to coculture, since there is increasing evidence that pancreas follows a developmental program relatively close to that of liver. When isolated mouse pancreas β cells (MIN6) were cocultured with HUVEC and MSC, a similar formation of cell condensate was observed (Fig. 3). To visualize the internal structure of the generated organoids, confocal microscopic analyses were performed with fluorescence-labeled cells. 3-D Z-stack images revealed that kusabira Orange (KO)-labeled MIN6 self-organized in 72 hr after transplantation to form islet-like tissues, whereas green fluorescence protein (EGFP)-labeled HUVEC formed a network structure covering the MIN6-derived islets inside the organoids. These results indicated a possibility that the operating principle found in liver might be extended to pancreas.

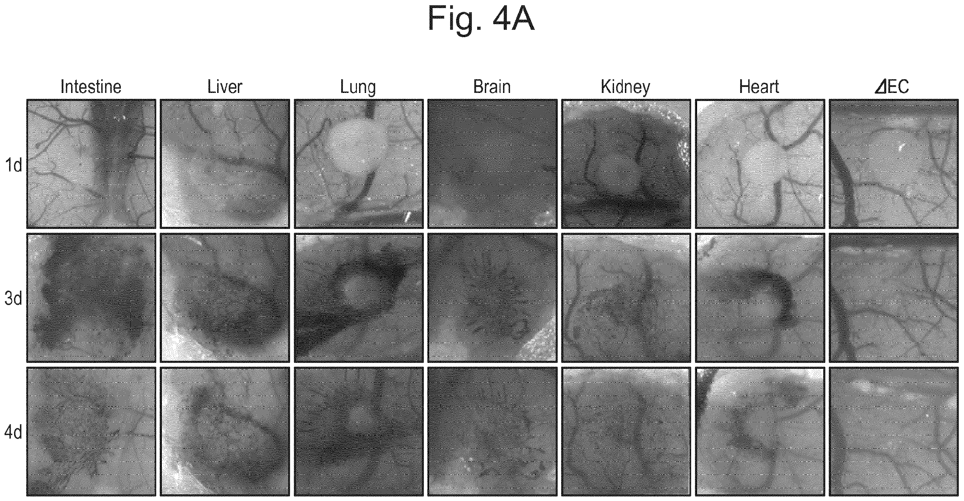

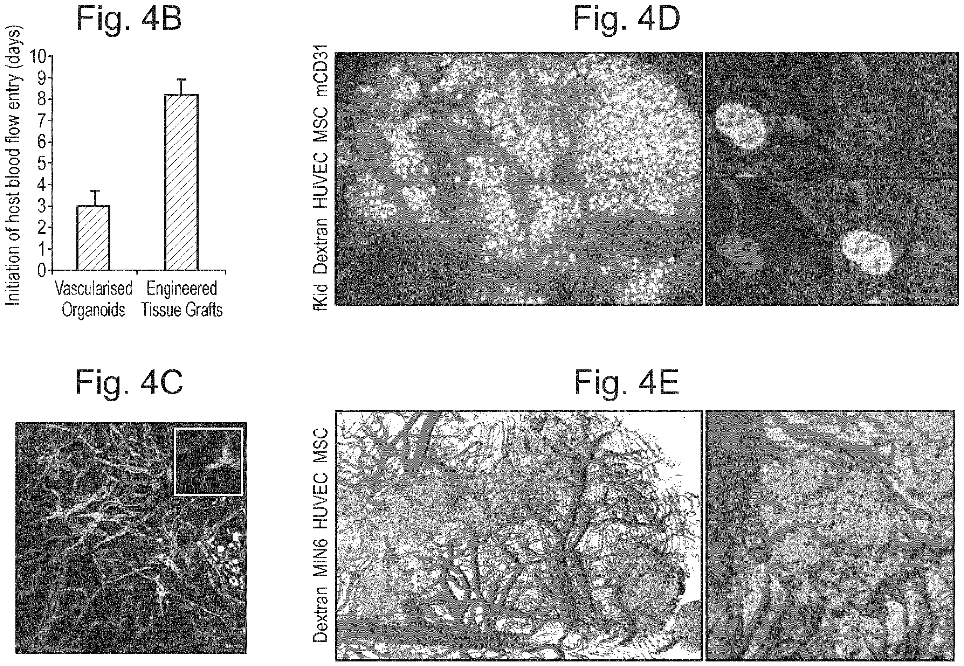

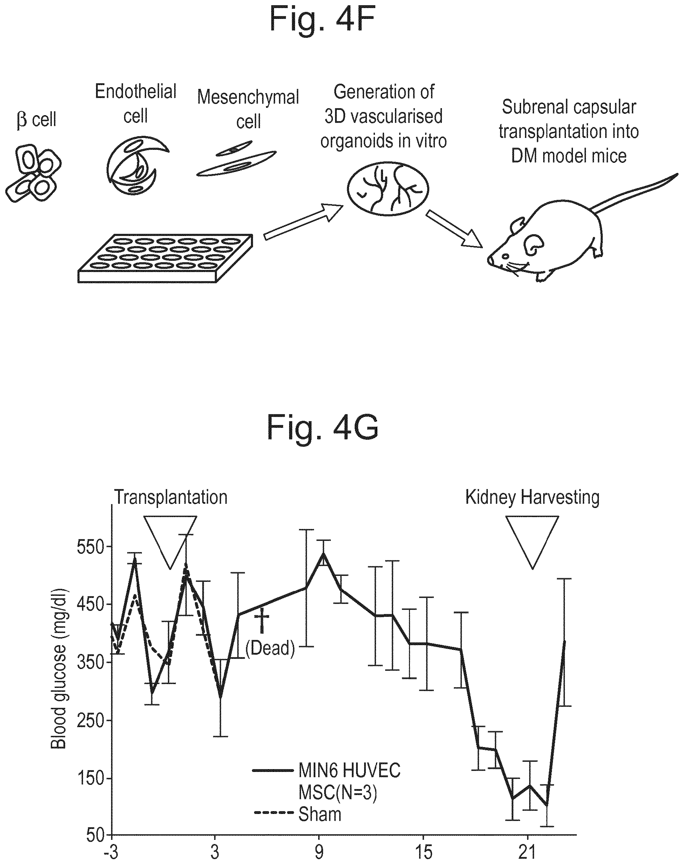

Next, to assess further versatility of this approach, multiple cells or tissue fragments (up to 200 µm) were isolated from embryonic or adult mice. Surprisingly. the directed and autonomic assembling phenomenon was retained in all the cell/tissue types tested, including pancreas, liver, intestine, lung, heart, kidney, brain, and even cancer (Fig. 3). Time-lapse imaging analyses revealed that both the embryonic and adult cells/tissues successfully resisted additional manipulations (including surgical transplantation) to form single 3D organoids autonomously (Fig. 3). Condensates as designed to contain cultured endothelial cells (HUVECs) turned out to permit a much more rapid perfusion with recipient circulation after transplantation (average perfusion time: -72 hr) compared with reliable conventional tissue engineering approaches (average perfusion time: ~192 hr). These results suggest that scaffold-free and self-assembly approaches are superior in terms of vasculogenesis (Fig. 4). Although the presence of endothelial cells is dispensable for the generation of condensates, the post-transplant outcomes are clearly disappointing in the absence of HUVECs because no signs of functional vascularization are observed *in vivo* (Fig. 4).

Interestingly, most of adult organ cell-derived condensates, although retaining functional vascularization, failed to reconstitute tissues resembling the original tissues after transplantation (Fig. 7). However, embryonic cell-derived condensates efficiently reconstituted functional tissue units through self-organization. For example, transplantation of embryonic kidney-derived organoids reconstituted glomerular-like microtissues with signs of blood filtration (Fig. 4D), whereas adult kidney- or lung-derived condensates failed to produce such tissues (Figs. 7 and 8). These results raise a question to the dominant paradigm in regenerative medicine that mature cell transplantation using cells directly differentiated from PSC might be effective for treating organ failures, because terminally differentiated cells have only poor ability to reconstitute functional tissues upon transplantation, even under well vascularized conditions.

Subsequently, pancreatic cells were selected for in-depth characterization. The transplantation of 3-D pancreatic organoids resulted in rapid (~48 hr) reperfusion and successful β cell engraftment. These were confirmed by live imaging analysis. After 14 days, the transplants developed islet-like structures (Fig. 4, E) with functional microvascular networks that connected to the recipient circulatory system (Fig. 4 C). Such blood perfusion was not recognized when condensates not containing vascular endothelial cells were transplanted (Fig. 9). The reconstituted islets directly connected to peripheral mouse blood vessels to be highly vascularized with a tight network of microvessels (Fig. 10). The capillary network in the islet in a living body is known to be approximately 5 times as dense as the capillary network surrounding exocrine secretion tissues. Consistent with this, intravital quantification of the functional vascular density showed that the capillary network was much denser (by 4.2 times) in the reconstituted islet-like tissues than in the areas surrounding the normal tissues (Fig.4, Fig. 9). Histological analysis also showed that the islet-like tissues had a structure resembling the adult islet, suggesting the reconstitution of a mature tissue via self-organization (Fig. 11). Further, to evaluate their therapeutic efficiency, *in vitro*-derived β cell organoids were transplanted into kidney subcapsule of type 1 fulminant diabetic model mouse. As the diabetic model, a transgenic mouse was used, namely a toxin receptor-mediated cell knockout (TREK) Tg mouse having a diphtheria toxin (DT) receptor cDNAtransgene in insulin promoter. While mice in non-transplantation group died at day 6 of DT administration-mediated induction of diabetes, those mice which received transplantation of β cell organoids maintained normal blood glucose levels and survived (Fig. 4, G). Thus, the applicability of the foregoing principle to other organ systems was demonstrated by experimentally recapitulating vascularization and reconstituting a functional three-dimensional tissue *in vivo.*

In the 1960s, aggregates of dissociated embryonic cells were shown to reconstitute tissues with a structure resembling that of the original tissue via self-organization. Once the required small numbers of various cells have aggregated to become capable of close interactions, individual cells are able to self-organize to form functional tissues *in vitro* (14). This classic knowledge about self-organization is capable of bringing about a technical revolution in the field of regenerative medicine which designs a principle for growing organs from PSC, one substantial challenge in this field. Now, this principle has been reinforced with observations of brain, optic cup, kidney and liver from PSC-derived cell aggregates by the present inventors and other researchers (15). In this context, one promising principle has been demonstrated by the present work. Briefly, in contrast to conventional methods each enabling the formation of only small-size condensates (aggregates), the principle under consideration ensures that starting with larger numbers of the desired cells/tissues, self-organized organoids can be designed via condensation. The condensates may be used for examining the subsequent self-organization capacity both *in vitro* and *in vivo.* In the foregoing study, rapid vasculogenesis and subsequent functionalization were evaluated by incorporating endothelial cells experimentally. For more precise reconstruction of tissues, evaluating the contribution of undeveloped supporting cells such as neurons is also an interesting topic for the present inventors and other research groups. Although further improvement is necessary for determining optimal conditions for self-organization of tissues of interest, it is believed that the culture principle described above not only provides a powerful tool for studying human biology and pathology using pluripotent stem cells but also enables realization of regenerative medicine of the next generation for currently untreatable patients by using *in vitro* grown, complex tissue structures.

### References

1. K. Matsumoto, H. Yoshitomi, J. Rossant, K. S. Zaret, Liver organogenesis promoted by endothelial cells prior to vascular function. Science 294, 559 (Oct 19, 2001).
2. T. Takebe et al., Self-organization of human hepatic organoid by recapitulating organogenesis in vitro. Transplant Proc 44, 1018 (May, 2012).
3. T. Takebe et al., Generation of a vascularized and functional human liver from an iPSC-derived organ bud transplant. Nature protocols 9, 396 (Feb, 2014).
4. T. Takebe et al., Vascularized and functional human liver from an iPSC-derived organ bud transplant. Nature 499, 481 (Jul 25, 2013).
5. M. Eiraku et al., Self-organizing optic-cup morphogenesis in three-dimensional culture. Nature 472, 51 (Apr 7, 2011).
6. T. Nakano et al., Self-formation of optic cups and storable stratified neural retina from human ESCs. Cell stem cell 10, 771 (Jun 14, 2012).
7. N. G. Dulyaninova, R. P. House, V. Betapudi, A. R. Bresnick, Myosin-IIA heavy-chain phosphorylation regulates the motility of MDA-MB-231 carcinoma cells. Molecular biology of the cell 18, 3144 (Aug, 2007).
8. J. W. Shin et al., Contractile forces sustain and polarize hematopoiesis from stem and progenitor cells. Cell stem cell 14, 81 (Jan 2, 2014).
9. A. F. Straight et al., Dissecting temporal and spatial control of cytokinesis with a myosin II Inhibitor. Science 299, 1743 (Mar 14, 2003).
10. E. Dona et al., Directional tissue migration through a self-generated chemokine gradient. Nature 503, 285 (Nov 14, 2013).
11. D. E. Discher, P. Janmey, Y. L. Wang, Tissue cells feel and respond to the stiffness of their substrate. Science 310, 1139 (Nov 18, 2005).
12. Z. Liu et al., Mechanical tugging force regulates the size of cell-cell junctions. Proceedings of the National Academy of Sciences of the United States of America 107, 9944 (Jun 1, 2010).
13. H. Y. Yoshikawa et al., Quantitative evaluation of mechanosensing of cells on dynamically tunable hydrogels. Journal of the American Chemical Society 133, 1367 (Feb 9, 2011).
14. M. Takeichi, Self-organization of animal tissues: cadherin-mediated processes. Developmental cell 21, 24 (Jul 19, 2011).
15. Y. Sasai, Cytosystems dynamics in self-organization of tissue architecture. Nature 493, 318 (Jan 17, 2013).

### Materials and Methods

### - Preparation of mesenchymal cells (MCs)

As for MCs, any of the following cells was used: cells isolated from human bone marrow, cells isolated from umbilical cord stroma (Wharton's sheath), cells isolated from human auricle, cells isolated from mouse bone marrow, human fibroblast cells or the like. The mesenchymal stem cells isolated from human bone marrow (hMSCs) that were mainly used in this experiment had been cultured using MSCGM^{™} BulletKit^{™} (Lonza PT-3001), a medium prepared exclusively for hMSC culture.

### - Preparation of various cells

After anesthetization with diethyl ether (Wako), the abdomens of C57BL/6-Tg (CAG-EGFP) mice (Nippon SLC) at days 12-17 of gestation were disinfected with 70% ethanol and incised to remove embryos. Brain, heart, lung, liver, metanephros or intestine was removed from the embryos. Brain, heart, lung, liver, kidney or intestine was also removed from C57BL/6-BALB/c RFP hairy mice 6 or more weeks of age (purchased from Anticancer Inc.). When cells isolated from these removed tissues were used, they were put in 200 µl of 0.05% Tryspin-EDTA (GIBCO) and incubated for 20 min at 37°C. Subsequently, the tissues were disrupted with a pipette and added to 4.8 ml of a medium. After centrifugation, medium was added and the number of cells was counted. Then, enzyme treatment was conducted to give single cells, which were subsequently used for coculture. When the removed tissues were to be used in a state of small tissues, the removed embryonic tissues were minced with scissors, put in 10 ml of 0.05% Tryspin-EDTA and shaken for 20 min at 37°C. After addition of medium, the resultant cells were passed through a 100 µm cell strainer and centrifuged. After centrifugation, medium was added for use in cell culture. The brain, heart, lung and kidney of the adult mice were minced with scissors and passed through a 100 µm cell strainer. The resultant flow-through was filtered with a 40 µm cell strainer. The cell mass remaining on this cell strainer was collected with medium for use in coculture of cells. With respect to the small intestine of adult mice, the contents were washed with physiological saline. The washed small intestine was cut lengthwise at intervals of 4 cm. The resultant sections were put in 2 mM EDTA, 0.5 mM DTT in PBS and shaken for 20 min at 37°C. Subsequently, the cells were passed through a 100 µm cell strainer, followed by addition of PBS. After centrifugation, the supernatant was suctioned and PBS was added for washing. Then the cells were centrifuged, and medium was added for use in cell culture.

With respect to normal umbilical vein endothelial cells (HUVECs), either cells isolated from the umbilical cords provided by maternal women at the time of delivery after informed consent or purchased cells were cultured in EGM^{™} BulletKit^{™} (Lonza CC-4133) through no more than 5 passages. Either type of the cells were fluorescence-labeled with retrovirus vector when necessary. HepG2 was cultured in DMEM supplemented with 10% FBS. Each type of cells were cultured in a 37°C, 5%CO₂ incubator.

### - Preparation of cell condensates for self-organization

On each well of a 24-well plate on which PA gel planar substrate was placed or to which Matrigel coating [Matrigel (BD) either in stock solution or as a mixture with medium at 1:1 was poured at 300 µl/well and left to stand in a 37°C, 5% CO₂ incubator for 10 min until it solidified] was applied, 2×10⁶ cells or more of multiple types in any combination (tissues isolated from embryo or adult, or KO-HepG2, HUVEC, etc.) as mixed with 2×10⁵ MSCs were seeded. In order to form small-sized cell condensates, 4×10³ cells or more of multiple types in any combination (tissues isolated from embryo or adult, or KO-HepG2, HUVEC) were mixed with 5×10³ or more MSCs and seeded. In either case, the cells were subsequently cultured in a 37°C incubator for a day. After seeding, chronological observation of cell coculture was performed with a stereomicroscope or a confocal microscope. There is no limitation of the cell types in "any combination". For example, cells derived from different tissues such as pancreas, liver, intestine, nerve, etc. may be mixed and used. Subsequently, an optimal composition for self-organization of an organ of interest could be used.

In experiments performing cell image analyses using plates on which PA gel planar substrate was placed, evaluation was made based on movies of the process of condensate formation as realized by seeding the two types of cells, HUVEC (2-4×10⁶ cells) and hMSC (2-4×10⁵ cells).

### - Preparation of PA gel planar substrate (uniform in-plane stiffness)

As a gel reaction solution, a 10 ml solution was prepared by mixing aqueous acrylamide solution (40% w/v, A4058, Sigma), aqueous bis-acrylamide solution (2% w/v, M1533, Sigma) and distilled water. In the process, the Young's modulus of the gel was adjusted by changing the mixing ratio of the individual solutions. The resultant reaction solution was bumped using a vacuum chamber. Then, 100 µl of APS (5 g/DW 50 ml, 01307-00, KANTO, 0.20 mm filtered) and 10 µl of TEMED (T9281, Sigma) were sequentially added to the reaction solution. Subsequently, the reaction solution (25 µl) was dripped onto a hydrophobic glass slide (S2112, MATSUNAMI) functionalized with dichlorodimethylsilane (DCDMS, D0358, TCI) and then a round glass coverslip (ϕ=12-25 mm, MATSUNAMI) treated with allytricholorosilane (ATCS, 107778-5g, Sigma) was placed on top to provide a sandwich structure, which was then left to stand for 30 min. Subsequently, distilled water was added to the sandwiched sample, which was then left to stand overnight. Then, the glass coverslip was peeled off from the glass slide, leaving a gel coat on the former. Phosphate buffer was added to the resultant glass coverslip, which was then left to stand for one day to remove the unreacted monomers. Table 1 below shows representative mixing ratios for the reaction solution and the Young's moduli of gels. The Young's moduli of gels were determined by nanoindentation measurements performed with an atomic force microscope (Nanowizard 3, JPK Instruments, Germany).

Coating of adhesion molecules (Matrigel or laminin) onto the PA gel surface was performed by the procedures described below. First, 0.2 mg/ml N-sulfosuccinimidyl-6-(4'-azido-2'-nitrophenylamino)hexanoate (Sulfo-SANPAH, 22589, Pierce) in 20 mM HEPES (pH 8.5) was dripped onto the PA gel substrate, followed by irradiation with a UV lamp (Z169633-1EA, Sigma) for 20 min. Subsequently, 4.4 mg/ml Matrigel solution [stock solution diluted 227-fold in 20 mM HEPES (pH 8.5), 354234, BD] or 10.0 mg/ml laminin solution [stock solution diluted 227-fold in 20 mM HEPES (pH 8.5), 354232, BD] was dripped in several milliliters onto the gel surface. The resultant gel was left to stand in a 37°C incubator for 16 hr. Finally, the PA gel was thoroughly rinsed with phosphate buffer to remove the uncrosslinked Matrigel or laminin.

**Table 1. Mixing Ratio for Solution and Young's Modulus in Representative Cases of Gel Synthesis**

| **Sample ID** | **Acrylamide 40% sol [mL]** | **Bis-acrylamide 2% sol [mL]** | **Distilled Water [mL]** | **Young' s modulus [kPa]** |
|---|---|---|---|---|
| S1 | 0. 75 | 0. 5 | 8. 75 | 1. 5 ± 0. 1 |
| S2 | 1. 25 | 0. 75 | 8 | 13. 6 ± 1. 2 |
| S3 | 1. 25 | 1. 125 | 7. 625 | 17. 0 ± 0. 3 |
| S4 | 2 | 1. 32 | 6. 68 | 54. 3 ± 1. 2 |
| S5 | 2 | 2. 4 | 5. 6 | 105 ± 33. 7 |

### [Example 2]

On each well of a 24-well plate to which Matrigel coating [Matrigel (BD) either in stock solution or as a mixture with medium at 1:1 was poured at 300 µl/well and left to stand in a 37°C, 5% CO₂ incubator for 10 min until it solidified] was applied, 2×10⁶ iPS cell-derived hepatic endoderm cells or human adult hepatocytes as mixed with 5×10⁵ human or mouse MSCs were seeded. In either case, the cells were subsequently cultured in a 37°C incubator for a day. After seeding, chronological observation of cell coculture was performed with a stereomicroscope or a confocal microscope. As shown in Fig. 13, even in the absence of vascular endothelial cells, cell condensates could be formed as long as mesenchymal cells were present.

### [Example 3]

### - Preparation of U-bottom PA gel

As a gel reaction solution, a 10 ml solution was prepared by mixing aqueous acrylamide solution (40% w/v, A4058, Sigma), aqueous bis-acrylamide solution (2% w/v, M1533, Sigma) and distilled water. In the process, the Young's modulus of the gel was adjusted by changing the mixing ratio of the individual solutions. This reaction solution (500 µl) was added to a 24-well tissue culture plate (353047, BD). Then, 0.5 µl of TEMED (T9281, Sigma) and 5 µl of APS (5 g/DW 50 ml, 01307-00, KANTO, 0.20 mm filtered) were added to the reaction solution in this order, immediately followed by thorough mixing. Then, the plate was left to stand on a 50°C hot plate for 15 min. Phosphate buffer was then added and the plate was left to stand for one day to remove the unreacted monomers. A cross section of the resultant U-bottom gel is shown in Fig. 12.

In cell condensate formation experiments using this gel (having a constant stiffness of about 30 kPa at depths of 3 microns and more), 2×10⁶ iPS cell-derived hepatic endoderm cells, 7×10⁵ HUVECs and 2×10⁵ human MSCs were mixed and seeded on each well of 24-well plate. Then, the cells were incubated in a 37°C incubator for one day. After seeding, chronological observation of cell coculture was performed with a stereomicroscope or a confocal microscope. The results revealed that cell condensates were formed (Fig. 14).

### [Example 4]

### (Methods and Results)

After anesthetization with diethyl ether (Wako), the abdomens of C57BL/6-Tg (CAG-EGFP) mice (Nippon SLC) at days 12.5 and 13.5 of gestation were disinfected with 70% ethanol and incised to remove embryos. Metanephros was removed from the embryos, put in 200 µl of 0.05% Tryspin-EDTA (GIBCO) and incubated for 20 min at 37°C. Subsequently, the tissues were disrupted with a pipette and added to 4.8 ml of a medium. After centrifugation, medium was added and the number of cells was counted. Then, enzyme treatment was conducted to give single cells, which were subsequently used for coculture. Thereafter, the cells were mixed with mesenchymal stem cells isolated from human bone marrow (hMSCs) and normal umbilical vein endothelial cells (HUVECs) and seeded on wells where a solution obtained by mixing Matrigel (the stock solution of Matrigel (BD) used in Example 2) and a medium for vascular endothelial cells (EGM BulletKit^{™}, Lonza CC-4133) at 1:1 had been solidified. In the case of a 24-well plate, cells in any types of combinations were seeded in each well for a total cell count of about 2×10⁶. The mixing ratio of embryonic renal cells, MSCs and HUVECs was 10:2:0.1-1 but this is not the sole case of the applicable mixing ratio. Thereafter, the cells were cultured in a 37°C incubator for one day. As a result, three-dimensional tissues formed autonomously. The result shown in the lower left panel of Fig. 16 were obtained from tissues formed by pellet culture. To prepare pellet tissues, the method described in Christodoulos Xinaris et al. (In Vivo Maturation of Functional Renal Organoids Formed from Embryonic Cell Suspensions. J Am Soc Nephrol. 2012 Nov; 23(11): 1857-1868.) was essentially adopted using a technique in which isolated cells as collected simultaneously were allowed to assemble in the bottom of a tube by centrifugal force to form tissues for transplantation.

The renal primordium formed was transplanted into the wombs of immunodeficiency mice. As a result of macroscopic observation, blood perfusion was recognized in two to three days after transplantation (Fig. 15, upper row). The white dotted lines in Fig. 15 indicate the transplantation areas. Scattered cells formed spherical, glomerular tissues at day 8 of transplantation (Fig. 15, bottom row). The results of fluorescence observation as shown in the left panel of Fig. 16 revealed that a great number of glomerular structures were formed by culturing on a support but that this was not the case when the conventional method (pellet transplantation group) was applied. The results of comprehensive gene expression analyses as shown in Fig. 16, right panel, revealed that the transplants at one month after transplantation had matured to a degree equivalent to that of 0-8 weeks after birth. As shown in the three left columns of Fig. 17, the results of electron microscopy targeting tissues at week 4 of transplantation revealed that the resulting tissues formed normal nephron structures comprising podocytes, slit membranes, endothelial cells, proximal tubules, mesangial cells and the like. As shown in the rightmost panel of Fig. 17, the results of immunostaining confirmed the presence of podocytes and slit membranes. Further, the results of fluorescence live observation as performed after administration of low molecular weight fluorescence dextran at week 3 of transplantation are also shown (Fig. 18). The tissues formed first flowed into blood vessels, were filtered inside glomeruli, and collected in proximal tubules, indicating that they had the primitive urine producing function of the kidney (Fig. 18). As described above, by transplanting into a living body the renal primordium artificially prepared according to the present invention, autonomous maturation could successfully be induced to prepare functional renal tissues.

### [Example 5]

### (Methods and Results)

Changes in the Young's Modulus of supports (see "preparation of PA gel planar substrate") with different stiffness conditions (Samples A, B and C) before (oblique lines) and after (solid black) Matrigel coating. It was shown that the stiffness conditions can be strictly controlled regardless of the presence or absence of the coating (Fig. 19). The gel substrate used in Example 5 was prepared according to the method described in Example 1.

### [Example 6]

### (Methods and Results)

Gels having multiple stiffness patterns providing different stiffness conditions could successfully be prepared on one substrate (Fig. 20). According to pattern 1, a gel with a hard central part was prepared and according to pattern 2, a gel with a less hard central part was prepared (Fig. 20, left panel). The right panel of Fig. 20 shows the results of measurement of stiffness conditions along the major axis, indicating that the intended stiffness conditions could be achieved.

Gel substrates having spatial patterns of stiffness were prepared by the method described below. As a gel reaction solution, a 10 ml solution was prepared by mixing aqueous acrylamide solution (40% w/v, A4058, Sigma), aqueous bis-acrylamide solution (2% w/v, M1533, Sigma) and distilled water. Subsequently, with light shielded, 50 mg of Irgacure 2959 (0.5 % w/v, DY15444, Ciba) was added and dissolved in a hot water bath at 37°C. The resultant reaction solution was bumped in a vacuum chamber. Then, 10 µl of this reaction solution was dripped onto a hydrophobic glass slide (S2112, MATSUNAMI) functionalized with dichlorodimethylsilane (DCDMS, D0358, TCI) and then a round glass coverslip (ϕ=12 mm, MATSUNAMI) treated with allytricholorosilane (ATCS, 107778-5g, Sigma) was placed on top to provide a sandwich structure. A photomask was placed on the sandwiched sample and irradiated with UV light at 254 nm. The photomask was made from acetyl cellulose (G254B, Agar) by printing with a laser printer (MC860, OKI). Using Adobe Photoshop as a mask pattern designer, a circular mask (12 mm o.d. and 2-4 mm i.d.) was prepared. For irradiation, a mercury lamp (C - HGFI, Nikon) was used as a light source and fiber optics was combined with a light projection tube to ensure uniform irradiation of the reaction solution. The time of UV irradiation was adjusted by minutes depending on the desired stiffness. Subsequently, distilled water was added to the sandwiched sample and the glass coverslip was peeled off from the glass slide, leaving a gel coat on the former. Phosphate buffer was added to the resultant glass coverslip, which was then left to stand for one day to remove the unreacted monomers. The Young's moduli of gels were determined by nanoindentation measurements performed with an atomic force microscope (Nanowizard 3, JPK Instruments, Germany). Coating of adhesion molecules (Matrigel or laminin) onto the PA gel surface was performed by the procedures described below. First, 0.2 mg/ml N-sulfosuccinimidyl-6-(4'-azido-2'-nitrophenylamino)hexanoate (Sulfo-SANPAH, 22589, Pierce) in 20 mM HEPES (pH 8.5) was dripped onto the PA gel substrate, followed by irradiation with a UV lamp (Z169633-1EA, Sigma) for 20 min. Subsequently, 1 ml of 4.4 mg/ml Matrigel solution [stock solution diluted 227-fold in 20 mM HEPES (pH 8.5), 354234, BD] or 10.0 mg/ml laminin solution [stock solution diluted 227-fold in 20 mM HEPES (pH 8.5), 354232, BD] was dripped in one milliliter onto the gel surface. The resultant gel was left to stand in a 37°C incubator for 16 hr. Finally, the PA gel was thoroughly rinsed with phosphate buffer to remove the uncrosslinked Matrigel or laminin.

Subsequently, iPS cell-derived hepatic endoderm cells, HUVECs and MSCs were mixed at a ratio of 10:7:2 and the mixture was seeded on the patterned gels to give a total cell count of about 2×10⁶ cells (Fig. 21). As a result, in the gel with pattern 1, cells gathered in the central hard area within 30 hr after seeding to rapidly form condensates in the gel with pattern 2, formation of condensates was recognized but the velocity of cell movement toward the central part was slightly delayed. It was therefore suggested that the optimal condition for the stiffness of the central part was 100 kPa.

### [Example 7]

### (Methods and Results)

A positive pattern was so designed that individual circular parts were hard and their periphery was soft (Fig. 22, left panel). A negative pattern was so designed that individual circular parts were soft and their periphery was hard (Fig. 22, right panel). Gel substrates with such multiple patterns of stiffness were prepared based on the technique described in Example 6 and by exposing a gel substrate (25 mm in diameter) through a photomask with a 4x4 pattern of circles (diameter: about 2 mm; center-to-center distance between circles: about 2.7 mm). It is believed that by using these patterned supports, cell condensates of any size may be formed at any place.

### INDUSTRIAL APPLICABILITY

The present invention is applicable in various fields including search for new drugs and evaluation of their efficacy, regenerative medicine, diagnosis of diseases and pathology, and production of useful substances.

## Claims

1. A method of preparing a cell condensate *in vitro,* comprising culturing a mixture of cells and/or tissues of a desired type and undifferentiated mesenchymal cells to form a cell condensate, wherein the mixture of cells and/or tissues of a desired type and undifferentiated mesenchymal cells is cultured on a gel-like support on which the undifferentiated mesenchymal cells are capable of contraction, wherein the gel-like support has a stiffness of a Young's modulus of 1-200 kPa, and wherein the cell condensate is formed autonomously.

2. The method of claim 1, wherein the culture is two-dimensional culture.

3. The method of claim 1 or 2, wherein the gel-like support is planar or the side of the gel-like support on which culture is performed has a U- or V-shaped cross-section.

4. The method of any one of claims 1 to 3, wherein the gel-like support is:
(a) planar and the stiffness of the central part of the gel-like support is greater than the stiffness of the peripheral part thereof; or
(b) planar and patterned and has one or more patterns in which the stiffness of the central part is greater than the stiffness of the peripheral part.

5. The method of any one of claims 1 to 3, wherein the gel-like support is:
(a) planar and the stiffness of the peripheral part of the gel-like support is greater than the stiffness of the central part thereof; or
(b) planar and patterned and has one or more patterns in which the stiffness of the peripheral part is greater than the stiffness of the central part.

6. The method of any one of claims 1 to 5, wherein the size of the cell condensate is 1 mm or more.

7. The method of any one of claims 1 to 6, wherein the mixture of cells and/or tissues of a desired type and undifferentiated mesenchymal cells is cultured without using scaffold materials.

8. The method of any one of claims 1 to 7, wherein the cells and/or tissues mixed with the undifferentiated mesenchymal cells are derived from liver, pancreas, intestine, lung, kidney, heart, brain or cancer.

9. The method of any one of claims 1 to 7 wherein the cells mixed with the undifferentiated mesenchymal cells are pluripotent cells.

10. The method of any one of claims 1 to 7, wherein the tissues mixed with the undifferentiated mesenchymal cells are tissues induced from pluripotent cells.

11. The method of claim 9 or 10, wherein the pluripotent cell is a pluripotent cell obtained by induction from reprogramming.

12. The method of any one of claims 1 to 11, wherein the cells and/or tissues of a desired type have a total cell count of 400,000 or more and the undifferentiated mesenchymal cells are 100,000 to 400,000 in number.

13. The method of any one of claims 1 to 12, wherein the gel-like support has a stiffness of a Young's modulus of 1-100 kPa.

14. The method of claim 13, wherein the gel-like support has a stiffness of a Young's modulus of 1-50 kPa.

15. A method of preparing a three-dimensional tissue structure, said method comprising the method of any one of claims 1 to 14 and further comprising allowing self-organization of the cell condensate.

## Patentansprüche

1. Verfahren zur Herstellung eines Zellkondensats *in vitro,* das die Kultivierung einer Mischung aus Zellen und/oder Geweben eines gewünschten Typs und undifferenzierten mesenchymalen Zellen zur Bildung eines Zellkondensats umfasst, wobei die Mischung aus Zellen und/oder Geweben eines gewünschten Typs und undifferenzierten mesenchymalen Zellen auf einem gelartigen Träger kultiviert wird, auf dem die undifferenzierten mesenchymalen Zellen zur Kontraktion fähig sind, wobei der gelartige Träger eine Steifigkeit von einem Young'schen Modul von 1 bis 200 kPa aufweist, und wobei das Zellkondensat selbständig gebildet wird.

2. Verfahren nach Anspruch 1, wobei die Kultur eine zweidimensionale Kultur ist.

3. Verfahren nach Anspruch 1 oder 2, wobei der gelartige Träger planar ist oder die Seite des gelartigen Trägers, auf der die Kultur durchgeführt wird, einen U- oder V-förmigen Querschnitt aufweist.

4. Verfahren nach einem der Ansprüche 1 bis 3, wobei der gelartige Träger:
(a) planar ist und die Steifigkeit des zentralen Teils des gelartigen Trägers größer ist als die Steifigkeit des peripheren Teils davon; oder
(b) planar und gemustert ist und ein oder mehrere Muster aufweist, bei denen die Steifigkeit des zentralen Teils größer ist als die Steifigkeit des peripheren Teils.

5. Verfahren nach einem der Ansprüche 1 bis 3, wobei der gelartige Träger:
(a) planar ist und die Steifigkeit des peripheren Teils des gelartigen Trägers größer ist als die Steifigkeit des zentralen Teils davon; oder
(b) planar und gemustert ist und ein oder mehrere Muster aufweist, bei denen die Steifigkeit des peripheren Teils größer ist als die Steifigkeit des zentralen Teils.

6. Verfahren nach einem der Ansprüche 1 bis 5, wobei die Größe des Zellkondensats 1 mm oder mehr beträgt.

7. Verfahren nach einem der Ansprüche 1 bis 6, wobei die Mischung aus Zellen und/oder Geweben eines gewünschten Typs und undifferenzierten mesenchymalen Zellen ohne Verwendung von Gerüstmaterialien kultiviert wird.

8. Verfahren nach einem der Ansprüche 1 bis 7, wobei die mit den undifferenzierten mesenchymalen Zellen gemischten Zellen und/oder Gewebe aus Leber, Pankreas, Darm, Lunge, Niere, Herz, Gehirn oder Krebs stammen.

9. Verfahren nach einem der Ansprüche 1 bis 7, wobei die mit den undifferenzierten mesenchymalen Zellen gemischten Zellen pluripotente Zellen sind.

10. Verfahren nach einem der Ansprüche 1 bis 7, wobei die mit den undifferenzierten mesenchymalen Zellen gemischten Gewebe Gewebe sind, die aus pluripotenten Zellen induziert wurden.

11. Verfahren nach Anspruch 9 oder 10, wobei die pluripotente Zelle eine pluripotente Zelle ist, die durch Induktion aus Reprogrammierung erhalten wurde.

12. Verfahren nach einem der Ansprüche 1 bis 11, wobei die Zellen und/oder Gewebe eines gewünschten Typs eine Gesamtzellzahl von 400.000 oder mehr aufweisen und die undifferenzierten mesenchymalen Zellen 100.000 bis 400.000 an der Zahl sind.

13. Verfahren nach einem der Ansprüche 1 bis 12, wobei der gelartige Träger eine Steifigkeit von einem Young'schen Modul von 1 bis 100 kPa aufweist.

14. Verfahren nach Anspruch 13, wobei der gelartige Träger eine Steifigkeit von einem Young'schen Modul von 1 bis 50 kPa aufweist.

15. Verfahren zur Herstellung einer dreidimensionalen Gewebestruktur, wobei das Verfahren das Verfahren nach einem der Ansprüche 1 bis 14 umfasst und ferner das Ermöglichen der Selbstorganisation des Zellkondensats umfasst.

## Revendications

1. Procédé de préparation d'un condensat cellulaire *in vitro,* comprenant la culture d'un mélange de cellules et/ou de tissus d'un type souhaité et de cellules mésenchymateuses indifférenciées pour former un condensat cellulaire, dans lequel le mélange de cellules et/ou de tissus d'un type souhaité et des cellules mésenchymateuses indifférenciées sont cultivées sur un support de type gel sur lequel les cellules mésenchymateuses indifférenciées sont capables de se contracter, dans lequel le support de type gel a une rigidité d'un module de Young de 1 à 200 kPa, et dans lequel le condensat cellulaire est formé de manière autonome.

2. Procédé selon la revendication 1, dans lequel la culture est une culture bidimensionnelle.

3. Procédé selon la revendication 1 ou 2, dans lequel le support de type gel est plan ou le côté du support de type gel sur lequel la culture est effectuée a une section transversale en forme de U ou de V.

4. Procédé selon l'une quelconque des revendications 1 à 3, dans lequel le support de type gel est :
(a) plan et la rigidité de la partie centrale du support de type gel est supérieure à la rigidité de la partie périphérique de celui-ci ; ou
(b) plan et à motifs et a un ou plusieurs motifs dans lesquels la rigidité de la partie centrale est supérieure à la rigidité de la partie périphérique.

5. Procédé selon l'une quelconque des revendications 1 à 3, dans lequel le support de type gel est :
(a) plan et la rigidité de la partie périphérique du support de type gel est supérieure à la rigidité de sa partie centrale ; ou
(b) plan et à motifs et a un ou plusieurs motifs dans lesquels la rigidité de la partie périphérique est supérieure à la rigidité de la partie centrale.

6. Procédé selon l'une quelconque des revendications 1 à 5, dans lequel la taille du condensat cellulaire est de 1 mm ou plus.

7. Procédé selon l'une quelconque des revendications 1 à 6, dans lequel le mélange de cellules et/ou de tissus d'un type souhaité et de cellules mésenchymateuses indifférenciées est cultivé sans utiliser de matériaux d'échafaudage.

8. Procédé selon l'une quelconque des revendications 1 à 7, dans lequel les cellules et/ou tissus mélangés aux cellules mésenchymateuses indifférenciées sont dérivés du foie, du pancréas, de l'intestin, du poumon, du rein, du cœur, du cerveau ou du cancer.

9. Procédé selon l'une quelconque des revendications 1 à 7, dans lequel les cellules mélangées aux cellules mésenchymateuses indifférenciées sont des cellules pluripotentes.

10. Procédé selon l'une quelconque des revendications 1 à 7, dans lequel les tissus mélangés aux cellules mésenchymateuses indifférenciées sont des tissus induits à partir de cellules pluripotentes.

11. Procédé selon la revendication 9 ou 10, dans lequel la cellule pluripotente est une cellule pluripotente obtenue par induction à partir d'une reprogrammation.

12. Procédé selon l'une quelconque des revendications 1 à 11, dans lequel les cellules et/ou les tissus d'un type souhaité ont un nombre total de cellules de 400 000 ou plus et les cellules mésenchymateuses indifférenciées sont au nombre de 100 000 à 400 000.

13. Procédé selon l'une quelconque des revendications 1 à 12, dans lequel le support de type gel a une rigidité d'un module de Young de 1 à 100 kPa.

14. Procédé selon la revendication 13, dans lequel le support de type gel a une rigidité d'un module de Young de 1 à 50 kPa.

15. Procédé de préparation d'une structure tissulaire tridimensionnelle, ledit procédé comprenant le procédé selon l'une quelconque des revendications 1 à 14 et comprenant en outre la permission de l'auto-organisation du condensat cellulaire.
